# EUROPEAN PATENT SPECIFICATION

(11) **EP 3 322 412 B1**
(45) Date of publication and mention of the grant of the patent: **07.04.2021**
(21) Application number: 16739475.8
(22) Date of filing: 15.07.2016
(51) Int. Cl.: A61K 31/137, A61K 31/4709, A61P 25/24, A61P 25/22, A61P 25/06, A61P 25/00, A61P 25/18, A61P 25/20, A61P 21/00, A61P 13/00, A61P 15/12, A61P 29/00, A61K 9/00, A61P 25/28, A61P 25/16

(54) **USE OF AMITRIPTYLINE FOR BLOCKING BRAIN HEMICHANNELS AND METHOD FOR POTENTIATING ITS EFFECT IN VIVO**
VERWENDUNG VON AMITRIPTYLIN ZUM BLOCKIEREN VON GEHIRN-HEMICHANNELS UND VERFAHREN ZUR VERSTÄRKUNG SEINER IN-VIVO-WIRKUNG
UTILISATION DE L'AMITRIPTYLINE POUR LE BLOCAGE DES DEMI-CANAUX DU CERVEAU ET PROCÉDÉ DE POTENTIALISATION DE SON EFFET IN VIVO

(30) Priority: 15.07.2015 EP 15290186
(43) Date of publication of application: 23.05.2018
(73) Proprietor: Theranexus, 69008 Lyon (FR)
(72) Inventor: JEANSON, Tiffany, 78430 Louveciennes (FR); CHARVERIAT, Mathieu, 92130 Issy les Moulineaux (FR); MOUTHON,Franck, 92100 Boulogne Billancourt (FR)
(74) Representative: Regimbeau
(86) International application number: PCT/EP2016/066986
(87) International publication number: WO 2017/009472

(56) References cited:
- WO-A1-2008/134325
- WO-A1-2013/188847
- WO-A1-2015/011246
- US-A1- 2011 172 188
- JEANSON T ET AL: "Connexin channel inhibitor promotes the anti-hyperalgesic effect of amitriptyline in sciatic nerve-ligated rats", EUROPEAN NEUROPSYCHOPHARMACOLOGY, vol. 24, no. Suppl. 2, October 2014 (2014-10), pages S225-S226, XP002752553, & 27TH CONGRESS OF THE EUROPEAN-COLLEGE-OF-NEUROPSYCHOPHARMACOLOG Y (ECNP); BERLIN, GERMANY; OCTOBER 18 -21, 2014
- PRAHARAJ SAMIR K ET AL: "Amitriptyline for clozapine-induced nocturnal enuresis and sialorrhoea.", BRITISH JOURNAL OF CLINICAL PHARMACOLOGY JAN 2007, vol. 63, no. 1, January 2007 (2007-01), pages 128-129, XP002762447, ISSN: 0306-5251
- DATABASE COMPENDEX [Online] ENGINEERING INFORMATION, INC., NEW YORK, NY, US; 30 January 2015 (2015-01-30), CONG W-N ET AL: "Amitriptyline improves motor function via enhanced neurotrophin signaling and mitochondrial functions in the murine N171-82Q Huntington disease model", XP002762448, Database accession no. E20150500483990 & JOURNAL OF BIOLOGICAL CHEMISTRY 20150130 AMERICAN SOCIETY FOR BIOCHEMISTRY AND MOLECULAR BIOLOGY INC. USA, vol. 290, no. 5, 30 January 2015 (2015-01-30), pages 2728-2743, DOI: 10.1074/JBC.M114.588608
- PICOLI CHRISTÈLE ET AL: "Human connexin channel specificity of classical and new gap junction inhibitors.", JOURNAL OF BIOMOLECULAR SCREENING DEC 2012, vol. 17, no. 10, December 2012 (2012-12), pages 1339-1347, ISSN: 1552-454X
- HANSEN DANIEL BLOCH ET AL: "Activation, permeability, and inhibition of astrocytic and neuronal large pore (hemi)channels.", THE JOURNAL OF BIOLOGICAL CHEMISTRY 19 SEP 2014, vol. 289, no. 38, 19 September 2014 (2014-09-19), pages 26058-26073, ISSN: 1083-351X

## Description

### Summary of the invention

The present invention relates to the use of amitriptyline as an inhibitor of connexin hemichannels (HC) in the Central Nervous System (CNS) in a combination product with another HC-blocking agent, mefloquine, for treating in particular neuropathic pain, neurodegenerative disorders, ischemic brain injury and inflammatory intestinal conditions. More generally, the invention provides a method to enhance the therapeutic effect of amitriptyline for its common indications.

### Background of the invention

Tricyclic antidepressants (TCAs) are chemical compounds discovered in the early 1950s and marketed later in the decade.

Many side effects are related to the antimuscarinic properties of the TCAs. These side effects include dry mouth, dry nose, blurry vision, lowered gastrointestinal motility or constipation, urinary retention, cognitive and/or memory impairment, and increased body temperature. Other side effects include drowsiness, anxiety, emotional blunting (apathy/anhedonia), confusion, restlessness, dizziness, akathisia, hypersensitivity, changes in appetite and weight, sweating, sexual dysfunction, muscle twitches, weakness, nausea and vomiting, hypotension, tachycardia, and rarely, irregular heart rhythms.

Side effects may be less troublesome if treatment is initiated with low doses and then gradually increased, although this may also delay the beneficial effects.

Amitriptyline is a tricyclic antidepressant that has been frequently recommended as first-line treatment for major depressive disorder (MDD), post-traumatic stress disorder (PTSD), generalized anxiety disorder (GAD), social anxiety disorder (SAD), panic disorder, neuropathic pain, resistant pain, fibromyalgia, chronic musculoskeletal pain, akynesia in Parkinson's disease, cataplexy, migraine, Parkinson's disease, vasomotor symptoms of menopause, nocturnal enuresis, premenstrual dysphoric disorder (PMDD), bipolar I disorder, bulimia, and obsessive-compulsive disorder (OCD) (Finnerup et al 2015).

However, amitriptyline, as the other TCAs, has a limited efficacy and is endowed with poorly tolerated side effects (Moore, 2015).

Jeanson et al., 2014, reports that it is possible to potentiate the therapeutic activity of amitriptyline by combining same with an inhibitor of the astroglial gap junctions (connexin Cx30 and Cx43). This co-treatment did however not reduce the neuroinflammatory markers associated with hyperalgesia.

In this context, the present inventors aimed at enhancing amitriptyline therapeutic effect without worsening (and even better reducing) its side effects.

Neuropathic pain, the main pathology for which the tricyclic antidepressant amitriptyline is indicated, implicates HC activity (Huang et al, 2012; O'Carroll et al, 2013). More precisely, recent studies pointed out the implication of the astroglial connexin 43 (Cx43) in neuropathic pain physiopathology. Connexins constitute a family of proteins involved in the intercellular coupling by establishing gap junctions, which allow the exchange of small ions and molecules (< 1kDa) between adjacent cells (Giaume et al, 2013). These transmembrane proteins can also assemble into hexameric hemichannels (also called connexon), serving as diffusional pathways for ions and small molecules, required for intra- and extra-cellular compartments communication (Orellana JA et al, 2012). Mefloquine efficiently blocks hemichannels composed of several connexins, e.g., Cx26 (Levit, 2015), Cx36 (Pizarro-Delgado, 2014), Cx46 and Cx50 (Srinivas, 2005), and Cx43 (Khawaja, 2011). Moreover, meclofenamic acid (MFA) is also referred to as a hemichannel blocker (Richter, 2014).

In preclinical models of neuropathic pain, both expression and function of Cx43 are increased, suggesting that Cx43 may be involved in this disease. This has been observed in peripheral as well as central models of neuropathic pain (Chen et al 2012, Ohara et al 2008). In addition, Cx43 blocking by pharmacologic or genetic approaches is related to antinociceptive effects (Huang et al, 2012), reinforcing the fact that Cx43 would be implicated in this disease.

In this context, the present inventors hypothesized that amitriptyline therapeutic effect is (at least partially) due to the modulation of HC activity. Therefore, they studied the *in vitro* and *in vivo* effect of amitriptyline on CNS hemichannels. They found that, surprisingly, amitriptyline is able to efficiently block HC activity *in vitro.*

Consequently, they propose to use amitriptyline to treat pathologies that are known to involve HC activity, such as neurodegenerative disorders, ischemic brain injury and inflammatory intestinal conditions.

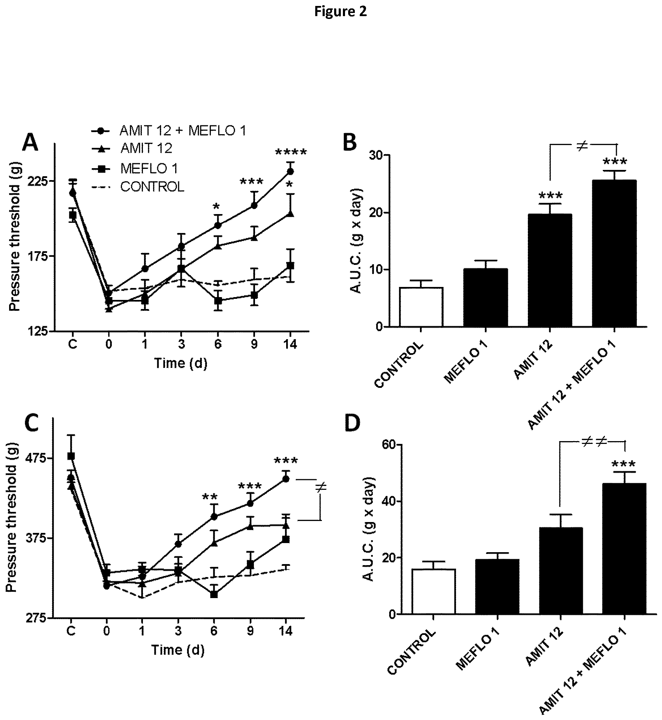

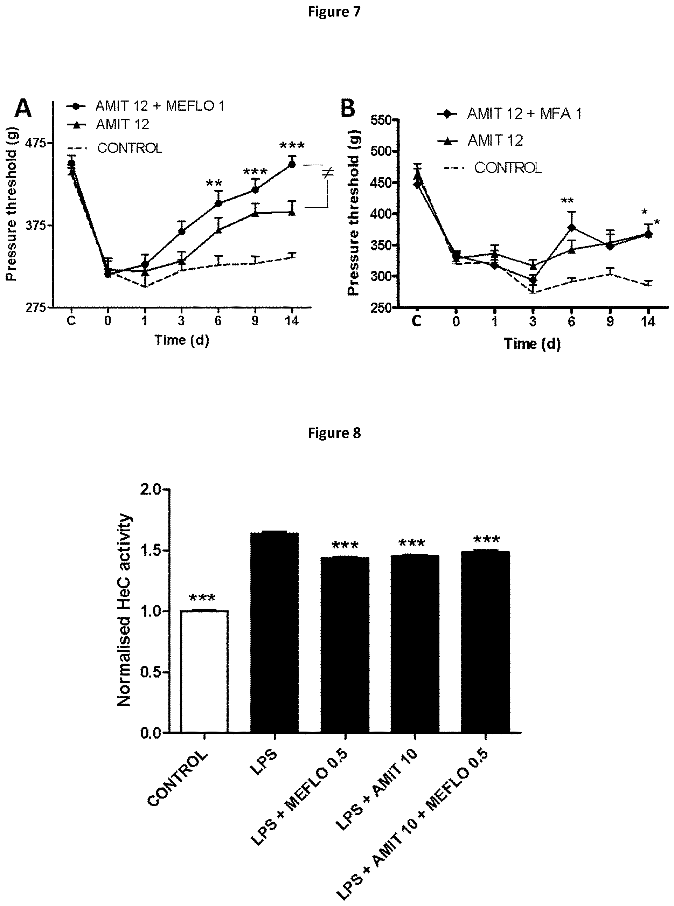

Looking for a way to enhance the therapeutic effect of amitriptyline, the inventors furthermore observed that a synergistic effect occurs between amitriptyline and mefloquine in animal models suffering from neuropathic pain, depression, sleep disorders. The inventors furthermore observed that this synergistic effect occurs between amitriptyline and mefloquine in neuro-inflammation, as model of neurodegenerative and psychiatric disorders (figures 2-5, 7, 10 and 13). Interestingly, this synergy appeared to be very specific for these two active principles, and occurred only *in vivo,* when amitriptyline is combined with a low dose of mefloquine (figures 7, 8 and 11).

The present invention therefore proposes to use a combination product containing amitriptyline and a low dose of mefloquine for preventing and/or treating subjects that are known to be sensitive to amitriptyline, namely subjects suffering from neuropathic pain, depression or sleep disorders.

### Detailed description of the invention

Amitriptyline has the following 2D formula:

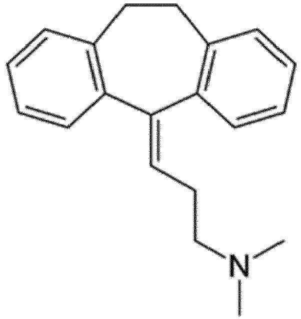

Its complete chemical name is 3-(5,6-dihydrodibenzo[2,1-b:2',1'-f][7]annulen-11-ylidene)-N,N-dimethylpropan-1-amine, and its CAS number is 50-48-6.

On a molecular point of view, amitriptyline inhibits the membrane pump mechanism responsible for the re-uptake of transmitter amines, such as norepinephrine and serotonin, thereby increasing their concentration at the synaptic clefts of the brain.

Amitriptyline acts as an antagonist of muscarinic receptors of acetylcholine and of alpha1 adrenergic receptors (Maj et al, 1982). Moreover, it is a blocker of calcium voltage-dependent channels as well as a potassium blocker (Gerner, 2003; Wooltorton, 1995). However, its main properties include the inhibition of the membrane pump mechanism responsible for the re-uptake of transmitter amines, such as norepinephrine and serotonin. Hence, it allows the latter amine concentration to increase at the synaptic clefts of the brain. Finally, as all tricyclic antidepressant, amitriptyline is also known to be an antagonist of histamine H1 receptor, hence inducing sedation.

Amitriptyline is mainly metabolized by demethylation by CYP2C19 forming nortriptyline (NT), and by hydroxylation, leading to the formation of E-10-hydroxyl-amitriptyline (EHAT) and Z-10-hydroxyl-amitriptyline (ZHAT). NT is further demethylated to desmethylnortriptyline (NNT) and hydroxylated to E-10-hydroxy-nortriptyline (EHNT) and Z-10-hydroxy-nortriptyline (ZHNT). The demethylation of amitriptyline and NT is mainly catalyzed by CYP2C19, with the participation of other CYP enzyme forms in higher drug concentrations (CYP1A2, 3A4, 2C9). CYP2D6 is the sole enzyme mediating the hydroxylation of amitriptyline (Rafael, 2008). More precisely, CYP3A4 plays a relatively minor role in amitriptyline clearance *in vivo.*

The term **"amitriptyline"** can designate any salt of amitriptyline (amitriptyline hydrochloride, or any other salts of amitriptyline). Amitriptyline precursors or metabolites (such as NT, NNT, EHAT, ZHAT, EHNT or ZHNT) which can be metabolized in the human body can be used, as well as its derivatives (for example, chemical derivatives resulting from one or several halogen substitutions and/or from addition of protective groups), which can be for example nortriptyline, desmethylnortriptyline, Z-10-hydroxynortriptyline, E-10-hydroxy-nortriptyline, Z-10-hydroxyl-amitriptyline, and E-10-hydroxylamitriptyline.

The term "amitriptyline" furthermore encompasses all pharmaceutically acceptable salts and all complexes (e.g., hydrates, solvates and clathrates) of said molecule. A particularly preferred salt of amitriptyline is amitriptyline hydrochloride. This salt is commercialized under different trademark names (Elavil, Tryptanol, Endep, Elatrol, Tryptizol, Trepiline, Laroxyl, Redomex) and is usually administered either orally or topically.

Amitriptyline is a widely used tricyclic antidepressant with sedative properties. More precisely, it is currently used for treating Major depressive disorder (MDD), post-traumatic stress disorder (PTSD), generalized anxiety disorder (GAD), social anxiety disorder (SAD), panic disorder, neuropathic pain, resistant pain, fibromyalgia, chronic musculoskeletal pain, akynesia in Parkinson's disease, cataplexy, migraine, Parkinson's disease, vasomotor symptoms of menopause, nocturnal enuresis in children, premenstrual dysphoric disorder (PMDD), bipolar I disorder, bulimia, and obsessive-compulsive disorder (OCD).

This drug is currently used for treating depression at 75 to 125 mg per day, for treating neuropathic peripheral pain in adults at 50 to 150 mg per day, and for treating or preventing nocturnal enuresis in children at 10 to 50 mg per day, depending on the children age.

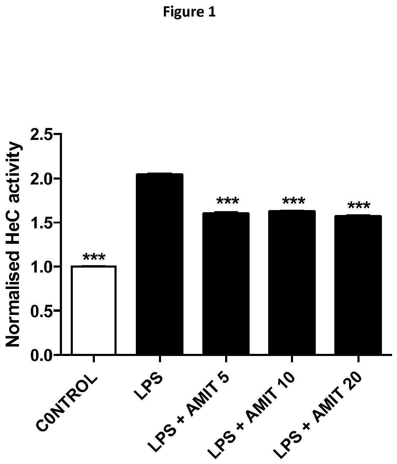

The present Inventors herein demonstrate that amitriptyline is able to efficiently block CNS hemichannel activity, in particular Cx43-based HC activity (cf. example 1.2.1. and figure 1).

This result was completely unexpected, since recent publications rather show an increase of the Cx43, both in expression and function, by antidepressants. Indeed the SSRI (Selective Serotonin Reuptake inhibitor) fluoxetine and the SNRI (Serotonine Norepinephrine Reuptake Inhibitor) duloxetine have been described as increasing Cx43 expression in the prefrontal cortex after 21-day treatment in rats (Fatemi et al, 2008; Sun et al, 2012). Amitriptyline itself has also been related to an increase of Cx43 expression, in mice cortical astrocytic cultures (Morioka et al, 2014). Consequently, it was thought so far that amitriptyline administration would trigger an increase of Cx43 expression and cellular coupling. Surprisingly, the results of the inventors rather show a reduction of one of Cx functions.

Connexins are protein subunits that oligomerize into hexamers called "connexons" or "hemichannels"(HC). Hemichannels allow direct communication between the cell cytoplasm and extracellular medium leading to the release of different molecules up to approximately 1 kDa in size such as ATP, NAD+ and glutamate.

There are 21 genes coding for different connexin isoforms in humans, and different combinations of connexin monomers involved in the composition of hemichannels have been described. In particular, the connexins 26 (Cx26), 36 (Cx36), 43 (Cx43), 32 (Cx32), 37 (Cx37) and 30 (Cx30) are expressed in human on cells of the Central or Peripheral Nervous System (Nakase & Naus, 2004; Baroja-Mazo, 2013; Orellana, 2013).

In the central nervous system, connexins are principally present in astrocytes which mainly express connexin 43 (Cx43). Therefore, Cx43 hemichannels are the most common hemichannels of the central nervous system.

The present disclosure thus teaches the use of amitriptyline, *in vitro* and *in vivo,* as an HC-blocking agent. In particular, it teaches the amitriptyline for use as a HC-blocking agent.

Hemichannels are usually closed and have a low open probability under physiological conditions. Negative membrane potentials, high concentrations of extracellular Ca²⁺ and intracellular H⁺ ions are different conditions which close hemichannels whereas positive membrane potentials and low extracellular Ca²⁺ open hemichannels. Moreover, pathological conditions drive hemichannel opening. For instance neuropathic pain, ischemic brain injury and neurodegenerative disorders, such as stroke, multiple sclerosis, amyotrophic lateral sclerosis, and Alzheimer's disease are characterized by hemichannel opening. This leads to the release of ATP and glutamate respectively implied in astrogliosis and neuronal excitability.

HC are composed by different connexins (Cx36, 43, 32, 26, 37, 30) and therefore expressed by cells from CNS or not (Baroja-Mazo, 2013; Orellana, 2013): Cx36 (neurons, microglia), Cx43 (astrocytes, microglia, corneal endothelial cells, heart, chondrocytes, smooth muscle cells), Cx26 (astrocytes, neurons, endolymphatic surface of cochlear supporting and epithelial cells), Cx32 (oligodendrocytes, microglia, neurons), Cx37 (neurons, monocytes, endothelial cells), Cx30 (endolymphatic surface of cochlear supporting and epithelial cell).

Currently different pharmacological tools can be used to block hemichannels. Unspecific connexin hemichannel blocker such as carbenoxolone or specific connexin hemichannel blockers as mimetic peptides, gap 26, gap 27. Lanthanum (La²⁺) can also be used to specifically block connexin hemichannels. Mefloquine efficiently blocks hemichannels composed of Cx26 (Levit, 2015), Cx36 (Pizarro-Delgado, 2014), Cx46 and Cx50 (Srinivas, 2005), Cx43 (Khawaja, 2011), with different selectivity towards those isoforms (Srinivas, 2005). MFA is also referred to as a hemichannel blocker (Richter, 2014).

As disclosed in the experimental part below, amitriptyline is particularly effective for inhibiting HC made of connexin Cx43.

The present disclosure teaches the *in vitro* use of amitriptyline as an anti-connexin agent. This agent can be used to inhibit HC made of the connexins selected in the group consisting of: Cx23, Cx25, Cx26, Cx30, Cx30.2, Cx30.3, Cx31, Cx31.1, Cx31.9, Cx32, Cx36, Cx37, Cx40, Cx40.1, Cx43, Cx45, Cx46, Cx47, Cx50, Cx59, and Cx62. Amitriptyline can be used in particular for blocking HC made of Cx43.

By **"blocking HC"** or **"inhibiting HC"** it is herein meant that diffusion of a dye (a fluorochrome, EtBr, etc.) is significantly reduced, or even abolished, in cells expressing HC. Said cells are for example cortical astrocytes activated by LPS, that mainly express Cx43. A reduction of 15%, preferably of 30% of the dye diffusion in the presence of the agent corresponds to a "blocking" effect according to the invention. Other methods have been proposed to identify potential blocking activity on HC, such as dye uptake (such as EtBr, LY, YoPro uptake), colorimetric Tietze method / colorimetric assay for glutamate release, HPLC analysis for glutamate release, bioluminescence ATP imaging for ATP release, luciferin/luciferase assay for ATP release, enzyme-linked fluorimetric assay for ATP or glutamate release, electrophysiological method, recording of HC currents (Giaume, 2013; Giaume, 2010).

Amitriptyline can be used for blocking Cx43.

Due to its anti-connexin activity, amitriptyline can be used for the treatment of a number of disorders and conditions known to involve connexin activity, for example cancers (WO 2006/134494), cardiovascular diseases (WO 2006/134494), wounds (WO 2006/134494), migraines (Durham and Garrett, 2009), epilepsy (Patel 2012), neurological conditions and neurodegenerative disorders (Takeuchi et al, 2011), ischemia (Davidson et al, 2013), drug-liver induced injury (Patel et al, 2012), infectious diseases (WO 2011/067607), cytotoxicity induced by chemotherapeutic agents (Tong X et al, 2013) and inflammatory disorders (WO 2006/134494).

Due to its HC blocking activity, amitriptyline can be used for the treatment of a number of disorders and conditions known to involve HC activity. These disorders and conditions include (but are not limited to):
- Neuropathic pain [Increased ATP release and glial activation related to increased HeC activity in neuropathic pain (Huang et al, 2012; O'Carroll et al, 2013)]
- Neurodegenerative disorders, such as stroke, multiple sclerosis, amyotrophic lateral sclerosis, and Alzheimer's disease [through the release of ATP and Glutamate via HeC (Orellana et al, *2010;* Koulakoff et al, 2012; Takeuchi et al, 2014)
- Ischemic brain injury (Davidson et al, 2013; Baroja-Mazo, 2013)
- Gastritis and peptic ulcer disease, inflammatory intestinal conditions, acute liver failure, cholestasis, hepatitis and steatosis, liver fibrosis and cirrhosis, infectious gastrointestinal pathologies, and gastrointestinal and liver cancer (Maes et al, 2015).
- Atherosclerosis (Baroja-Mazo, 2013)
- Optic nerve disease (Zhang, 2014)
- Bacterial and viral infections (Vega, 2013)
- Lens pathologies such as cataract (congenital cataract, intumescent cataract, early cortical cataract, mature cortical cataract, hypermature cortical cataract, morgagnian cataract, anterior subcapsular cataract, posterior subcapsular cataract, or nuclear cataract) which are associated with aberrant HC function in certain Cx mutation (Beyer, 2014; Mandal, 2015 ; Rhodes, 2009)
- Oculodentodigital syndrome or oculodentodigital dysplasia (ODDD), which is a mostly autosomal dominant disease caused by mutations in the Cx43 gene which is located on chromosome 6 (q21-q23.2) (certain mutations lead to an increase of the HC function and reduction of abnormal HC over-activity by HC inhibitor may treat some symptoms of ODDD) (Avshalumova, 2014; Paznekas, 2009 ; De Bock 2013)
- Optical neuropathy (e.g., due to demyelinating, non-arteritic, ischemic, arteritic inflammatory, infiltrative, compressive, toxic/nutritional, hereditary, traumatic, paraneoplastic disorders or to radiation) (Behbehani, 2007 ; Y. S. Chen, 2013; Danesh-Meyer, 2012)
- Keratitis (and hystrix-like) ichthyosis deafness (KID/HID) syndrome (aberrant over activity of HC is associated to this syndrome and reduction of this aberrant over activity may treat these type of hearing loss syndromes) (Jan, 2004 ; Sanchez, 2014)
- Clouston syndrome or hidrotic ectodermal dysplasia (HED) (which is associated with aberrant HC function in certain Cx mutation) (Babou, 2015)
- Cerebral hypoxia-ischemia (asphyxia) occurring in the fetus and newborn infant (reduction of hemichannel function was associated with improved recovery of brain activity, reduced seizure activity, and a more rapid return to fetal sleep state cycling, , following ischemia in the near-term fetal) (Vannucci, 2000 ; Davidson 2001)
- Pelizaeus-Merzbacher's disease (PMD) and related leukodystrophies (which are associated with aberrant HC function in certain Cx mutation) (Hobson, 2012 ; Diekmann, 2010)
- Juvenile Neuronal Ceroid Lipofuscinosis (JNCL) or Juvenile Batten Disease (which are associated with aberrant HC function in certain Cx mutation) (Wang, 2012 ; Burkovetskaya, 2014)

Amitriptyline can be used for the prevention and/or the treatment of neurodegenerative disorders, such as stroke, multiple sclerosis, amyotrophic lateral sclerosis, and Alzheimer's disease, or of ischemic brain injury.

Amitriptyline can be used for the prevention and/or the treatment of gastritis and peptic ulcer disease, inflammatory intestinal conditions, acute liver failure, cholestasis, hepatitis and steatosis, liver fibrosis and cirrhosis, infectious gastrointestinal pathologies, and gastrointestinal and liver cancer.

Amitriptyline can be used for the prevention and/or the treatment of lens pathologies such as cataract (congenital cataract, intumescent cataract, early cortical cataract, mature cortical cataract, hypermature cortical cataract, morgagnian cataract, anterior subcapsular cataract, posterior subcapsular cataract, or nuclear cataract), oculodentodigital syndrome or oculodentodigital dysplasia (ODDD), optical neuropathy, keratitis (and hystrix-like) ichthyosis deafness (KID/HID) syndrome, clouston syndrome or hidrotic ectodermal dysplasia (HED), cerebral hypoxia-ischemia (asphyxia) occurring in the fetus and newborn infant, Pelizaeus-Merzbacher's disease (PMD) and related leukodystrophies or Juvenile Neuronal Ceroid Lipofuscinosis (JNCL) or Juvenile Batten Disease.

In these indications, amitriptyline could be used at the previously disclosed doses, e.g., between 1 and 150 mg per day for an adult subject, and between 1 and 50 mg per day for a child.

As mentioned previously, amitriptyline has a limited efficacy and is endowed with poorly tolerated side effects (Moore, 2015). The present inventors therefore aimed at enhancing the therapeutic effect of amitriptyline while reducing its side effects on the treated subjects.

They found that a synergistic effect occurs between amitriptyline and mefloquine in animal models suffering from neuropathic pain, depression, sleep disorders or neuro-inflammation, as models of neurodegenerative and psychiatric disorders (see figures 2-5, 7, 10 and 13). Interestingly, this synergy is very specific, as it was not observed when amitriptyline was combined with a more efficient HC-blocking agent (meclofenamic acid, see figures 7 and 11), or with another HC-blocking agent (niflumic acid, see figure 11).

Moreover, this synergy was only observed *in vivo*: combining amitriptyline with mefloquine does not enhance its HC blocking activity *in vitro* in activated cortical astrocytic cells expressing Cx43. This suggests that mefloquine-mediated potentiation of amitriptyline *in vivo* likely involves other mechanism(s) than HC blocking.

Mefloquine is a 4-quinolinemethanol derivative with the specific chemical name of (R*, S*)-(±)-α-2-piperidinyl-2,8-bis (trifluoromethyl)-4-quinolinemethanol. Its CAS number is 53230-10-7. Its 2D formula is as follows:

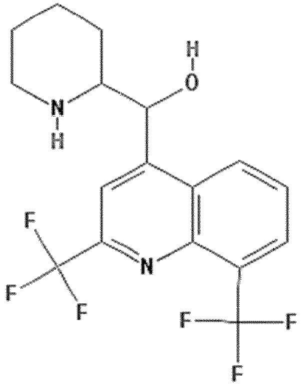

Mefloquine is metabolised by CYP3A4 to one major pharmacologically inactive metabolite, carboxymefloquine (2,8-bis-trifluoromethyl-4-quinoline carboxylic acid) and to a lesser extent to hydroxymefloquine (Fontaine, 2000).

The main metabolite is 2,8-bistrifluoromethyl-4-quinoline carboxylic acid. The carboxylic acid metabolite appeared in plasma 2 to 4 hours after a single oral dose. Maximum plasma concentrations, which were about 50% higher than those of mefloquine, were reached after 2 weeks. Thereafter, plasma levels of the main metabolite and mefloquine declined at a similar rate. The area under the plasma concentration-time curve (AUC) of the main metabolite was 3 to 5 times larger than that of the parent drug. The other metabolite, hydroxymefloquine, is present in minute quantities only.

Mefloquine hydrochloride tablets are indicated for the prevention or the treatment of mild to moderate acute malaria caused by mefloquine-susceptible strains of *P. falciparum* (both chloroquine-susceptible and resistant strains) or by *Plasmodium vivax.*

The term **"mefloquine"** further encompasses all pharmaceutically acceptable salts and all complexes (e.g., hydrates, solvates and clathrates), of said molecule. Prodrugs and derivatives of said molecule (for example carboxy-mefloquine (2,8-bis-trifluoromethyl-4-quinoline carboxylic acid) and hydroxyl-mefloquine (Fontaine, 2000)) can be used. A particularly preferred salt of mefloquine is mefloquine hydrochloride. This salt is commercialized under the brand names Lariam, Mephaquin or Mefliam, and is orally administered.

As the other HC-blocking agent MFA, mefloquine has already been proposed as treatment in neuropathic pain. Yet, it failed to show any effect in *in vivo* models *(Izzo, 1991; Xu, 2014).*

Conventional doses of mefloquine (Lariam) are of about 1250 mg in a single dose for treating a human adult suffering from malaria, whereas it is of about 250 mg per week for malaria prophylaxis. Children (weighting less than 45 kg) can be administered 20-25 mg/kg in a single dosed for malaria treatment, and 5mg/kg per week for malaria prophylaxis.

Mefloquine has four different stereoisomers. This drug is currently manufactured and sold as a racemate of the erythro-(*R,S*)- and erythro-(*S,R*)-enantiomers. Yet, the term **"mefloquine"** used in the combination product of the invention can be each enantiomer (either the (*R,S*) or the (*S,R*)) or stereoisomeric mixtures (racemates). The diastereoisomer threo-mefloquine can also be used (Bermudez, 2012; Muller, 2013).

In the present invention, any stereoisomer of mefloquine (such as S-erythro-mefloquine, R-erythro-mefloquine, S-threo-mefloquine or R-threo-mefloquine) can be used to potentiate the activity of the amitriptyline antidepressant.

Molecules having partial structural identity with mefloquine can furthermore be used. These molecules are for example quinoline methanol, more precisely quinine and quinidine, which are antimalarial agents isolated from the bark of the Cinchona tree. One can also cite chloroquine, primaquine, tafenoquine, pamaquine, amodiaquine, pentaquine, isopentaquine, quinocide, elubaquine, and bulaquine.

The term **"potentiate"** in this case means that mefloquine significantly increases the therapeutic effects of amitriptyline. Thus, the combination of mefloquine with amitriptyline makes it possible to reduce the doses of said psychotropic drug and therefore to limit the adverse effects of said psychotropic drug, and/or to obtain a stronger therapeutic effect without increasing the dose of said psychotropic drug.

The potentiating effects of mefloquine can be achieved by administrating mefloquine (a salt thereof, or an enantiomer thereof) to a subject, either before, at the same time, of after administration of amitriptyline to said subject.

Consequently, the present invention describes a method for treating a subject with psychiatric and/or neurodegenerative disorders, comprising the administration to said subject of a) mefloquine (a salt thereof or an enantiomer thereof) and b) amitriptyline (or a salt, thereof), in which said compounds a) and b) are administered simultaneously, separately or spread out over time.

Importantly, a low dose of mefloquine is sufficient to enhance the therapeutic effect of amitriptyline. By **"low dose of mefloquine",** it is herein meant a dose that is not able, on its own, to induce any therapeutic effect in the treated subject. Said dose is therefore typically lower than those currently approved for preventing or treating malaria. This low dose is preferably comprised between 10 µg and 50 mg per day for an adult subject. More preferably it is comprised between 100 µg and 25 mg per day, even more preferably between 500 µg and 20 mg per day, even more preferably between 1 mg and 25 mg per day, and most preferably between 1 mg and 10 mg per day for an adult subject. These doses have obviously to be adjusted for the children.

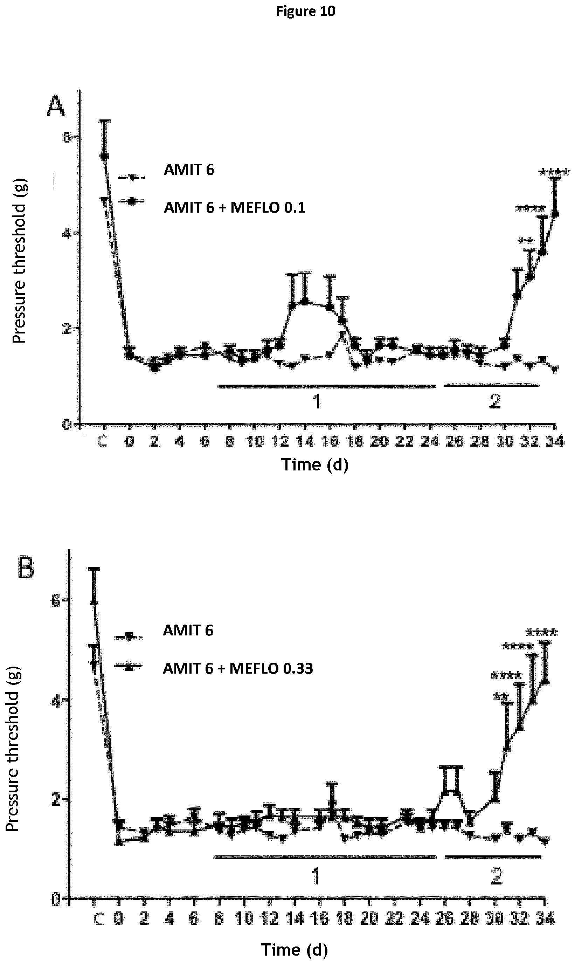

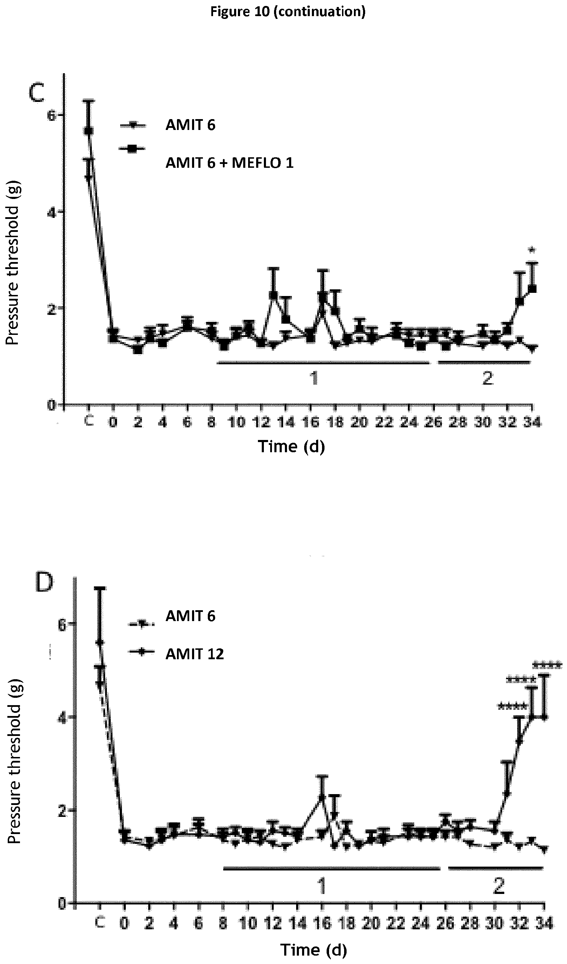

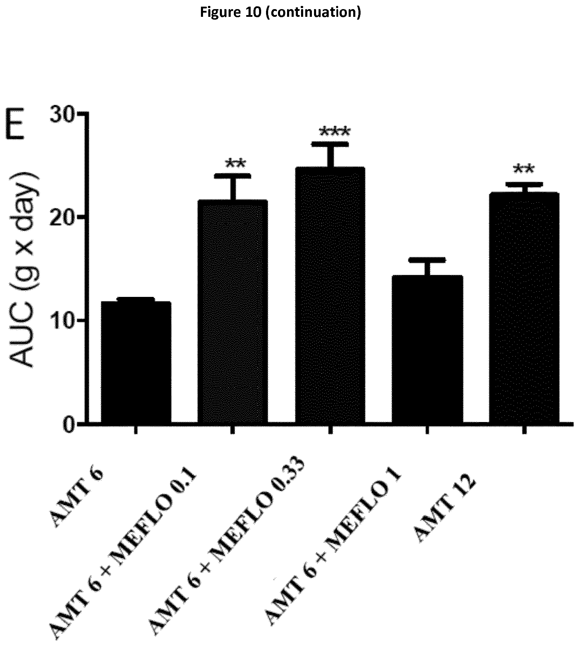

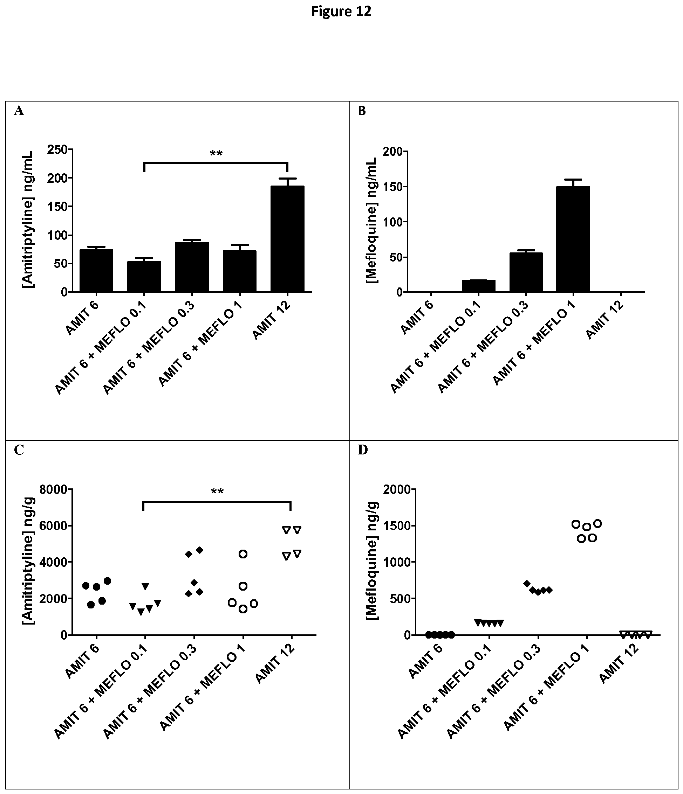

The inventors demonstrated that the doses of mefloquine inducing optimal potentiation of amitriptyline lead to plasma exposure of about 10% of the plasma exposure obtained with the doses of mefloquine that are conventionally used in human (usually 1000 to 2000 ng / mL). Higher doses of mefloquine, ie closer to those conventionally used, are less effective for potentiating amitriptyline (figures 10 and 12).

For example, Margineanu and Klitgaard, 2006, describes an *in vitro* inhibitory effect of connexins with a mefloquine brain concentration between 100 and 200 µM. In contrast, the inventors demonstrated that the *in vivo* potentiation of amitriptyline is optimal with mefloquine concentrations in plasma and brain of the micromolar scale, that is 100x less (figure 12).

Subjects needing this treatment may have psychiatric, neurologic and/or neurodegenerative disorders that are known to be treated by amitriptyline, for example those included in the group consisting of: Major depressive disorder (MDD), post-traumatic stress disorder (PTSD), anxiety, generalized anxiety disorder (GAD), social anxiety disorder (SAD), panic disorder, neuropathic pain, resistant pain, fibromyalgia, chronic musculoskeletal pain, akynesia in Parkinson's disease, cataplexy, migraine, nocturnal enuresis, nocturnal enuresis in children, nocturnal terrors, breathing disorders, Parkinson's disease, vasomotor symptoms of menopause, premenstrual dysphoric disorder (PMDD), restless syndrome (Willis-Ekbom disease), hypersalivation (drooling, hypersialorrhea), hypersalivation (drooling, hypersialorrhea) in amyotrophic lateral sclerosis, bipolar I disorder, bulimia, and obsessive-compulsive disorder (OCD).

More precisely, said subjects may suffer from different kinds of pain, for example post herpetic neuropathic pain, diabetic neuropathic pain, post chirurgical neuropathic pain, post chemotherapy neuropathic pain, post-stroke neuropathic pain, post cancer treatment neuropathic pain or post chemoprophylaxis neuropathic pain, post-HIV neuropathic pain, chronic back neuropathic pain, low back neuropathic pain, traumatic neuropathy, neuropathic pain related to multiple sclerosis disease or to other immune diseases, or neuropathic pain induced by spinal cord injury, that belong to the same nosological entity as neuropathic pain (Campbell, 2006; Pasero, 2004).

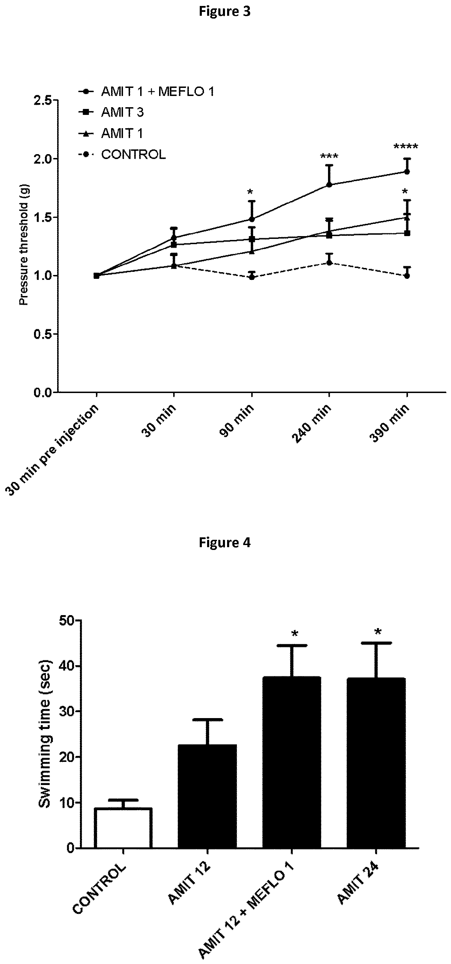

The inventors demonstrated the synergistic effect of the combination product of the invention on mice subjected to Forced Swim Test (FST) (figure 4). This test has been proposed as model for many neurologic disorders:
- Major depression disorder (Yankelevitch-Yahav, 2015)
- Bipolar disorder (van Enkhuizen, 2015)
- Fatigue (Guo, 2015)
- Depression (associated with neuropathic pain) (EIBatsh, 2015)
- Negative symptoms in schizophrenia (Mouri, 2007; Nabeshima, 2006; Russo, 2013)
- Negative effect of binge alcohol drinking in schizophrenia (Lee, 2015)
- Depression in Parkinson disease and in dementia with Lewy bodies (Weinstock, 2003)

Thus, the combination product of the invention can be efficiently used to prevent and/or treat any of the above-mentioned disorders.

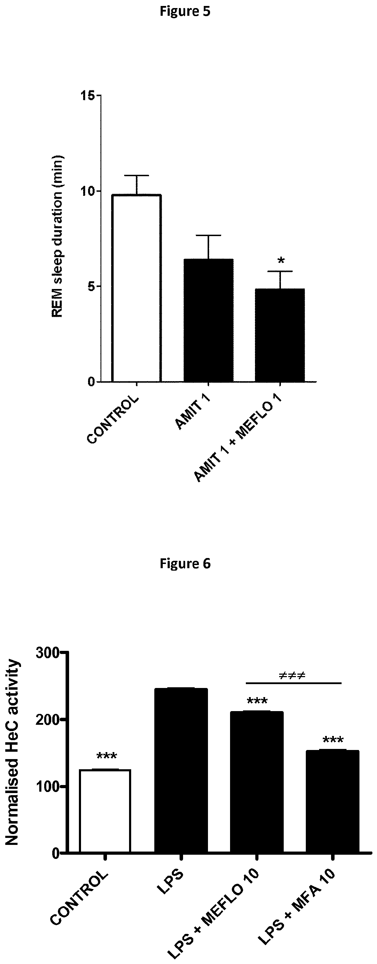

Furthermore, the inventors demonstrated the synergistic effect of the combination product of the invention on the REM sleep duration of mice (figure 5). This biomarker has been associated with many neurologic disorders:
- Major depressive disorder (MDD) (Palagini, 2013)
- Insomnia (comorbidity with depression) (Staner, 2010)
- Idiopathic REM sleep disorders (Postuma, 2015)
- Sleep bruxism (Sahin Onat, 2014)
- Sleep disorder in fibromyalgia (Roizenblatt, 2011)
- Sleep apnea (Musa, 1988)
- Multiple sclerosis (Veauthier, 2015)
- Neurodegenerative disorders (Parkinson's disease, Lewy body dementia, Alzheimer's disease, multiple system atrophy) (Ferini-Strambi, 2014; Ferman, 1999)

Thus, the combination product of the invention can be efficiently used to prevent and / or treat any of the above-mentioned disorders.

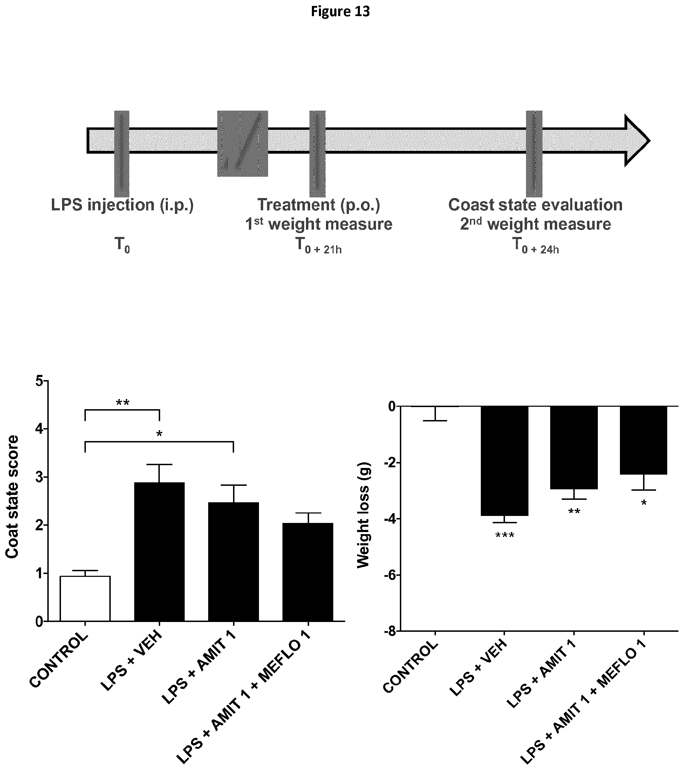

Furthermore, the inventors demonstrated the synergistic effect of the combination product of the invention on neuro-inflammation using the LPS model on mice (figure 13). This model has been widely used to characterize the neuro-inflammation associated with many neurodegenerative disorders (Qin *et al.,* 2010; Réus *et al.,* 2015) :
- Alzheimer's disease
- Parkinson's disease
- Huntington's disease
- Multiple sclerosis
- Amyotrophic lateral sclerosis;
and psychiatric disorders:
- Stress
- Depression
- Bipolar disorders
- Schizophrenia
- Autism, (Qin *et al.,* 2010; Réus *et al.,* 2015).

Thus, the combination product of the invention can be efficiently used to prevent and / or treat any of the above-mentioned disorders, including Major depressive disorder (MDD), post-traumatic stress disorder (PTSD), anxiety, generalized anxiety disorder (GAD), social anxiety disorder (SAD), panic disorder, neuropathic pain, resistant pain, fibromyalgia, chronic musculoskeletal pain, akynesia in Parkinson's disease, cataplexy, migraine, nocturnal enuresis, nocturnal enuresis in children, nocturnal terrors, breathing disorders, insomnia, Parkinson's disease, vasomotor symptoms of menopause, premenstrual dysphoric disorder (PMDD), restless syndrome (Willis-Ekbom disease), hypersalivation (drooling, hypersialorrhea), hypersalivation (drooling, hypersialorrhea) in amyotrophic lateral sclerosis, bipolar I disorder, bulimia, obsessive-compulsive disorder (OCD), fatigue, Alzheimer's disease, Huntington's disease, multiple sclerosis, amyotrophic lateral sclerosis, Lewy body dementia, multiple system atrophy, stress, depression, depression in Parkinson disease and in dementia with Lewy bodies, schizophrenia, autism, REM sleep disorders, idiopathic REM sleep disorders, sleep bruxism, sleep disorders in fibromyalgia, sleep apnea, fibrositis.

In the case of a simultaneous use, the two active principles of the treatment of the invention are administered to the subject simultaneously. According to this embodiment of the present invention, the two active principles can be packaged together, in the form of a mixture, or separately, then mixed extemporaneously before being administered together to the patient.

In a preferred embodiment, the two active principles are included in a solution, a capsule, a tablet, a syrup, a paste, a gel, a quick-disintegrating tablet, a delayed-released tablet, a microdose tablet, an ointment, a cream, a powder, an aerosolized spray, lozenge, etc. so as to be orally administered.

In another preferred embodiment, the two active principles can be included in a cream, an ointment, a spray, a paste, an adhesive patch, etc. so as to be topically applied.

Alternatively, the two active principles of the treatment of the invention can be administered separately. In particular, their routes of administration may be different. Their administration can also be performed at different sites. In this embodiment, the two active principles can be administered simultaneously, sequentially or spaced apart over time, for example the same day (few hours apart) or at an interval of one day, one week or one month.

In case of separate administration, mefloquine can be administered orally, and amitriptyline can be administered topically, in any of the forms disclosed above. Alternatively, the two active principles can be administered orally (but separately) in any of the forms disclosed above. Also, the two active principles can be provided topically in any of the forms disclosed above.

Since mefloquine potentiates the effects of amitriptyline, it can advantageously be used for reducing the doses of said psychotropic drug, thereby limiting its adverse effects, and/or reducing the risks of failure and withdrawal.

The effective equivalent dose of amitriptyline, *i.e.,* the amitriptyline dose that, when administered in combination with mefloquine, induces a physiological effect or a pharmacological signature similar or identical to that of amitriptyline alone administered at the active pharmacological dose, is about 0.5. In other words, it is possible to reduce the dose of amitriptyline by 2 folds when combined with mefloquine. The therapeutic effect of this half dose (combined with mefloquine) will be similar to the therapeutic effect of the complete dose of amitriptyline alone.

By **"subject"** it is herein meant any animal or human beings. Said subject is preferably a mammal, more preferably a human.

The present invention pertains to a composition, especially a pharmaceutical composition, comprising mefloquine and amitriptyline. This composition is preferably formulated for patients suffering from the psychiatric and/or neurodegenerative disorders disclosed above. In addition to mefloquine and amitriptyline, the composition can comprise any pharmaceutical vehicle, stabilizer, adjuvant and the like as frequently used in the art.

Examples of pharmaceutically acceptable vehicles include, but are not limited to: water; aqueous vehicles such as, but not limited to, sodium chloride solution, Ringer's solution, dextrose solution, dextrose and sodium chloride solution, and lactated Ringer's solution; water-miscible vehicles such as, but not limited to, ethyl alcohol, polyethylene glycol, and polypropylene glycol; and nonaqueous vehicles such as, but not limited to, corn oil, cottonseed oil, peanut oil, sesame oil, ethyl oleate, isopropyl myristate, and benzyl benzoate.

As mentioned above, the composition of the invention is preferably formulated for oral administration (including buccal cavity or sublingually) or topical administration. Other interesting formulations include formulations for intraperitoneal (i.p.), intravenous (i.v.), subcutaneous (s.c.), intramuscular (i.m.), transcutaneous, transdermal, intrathecal and intracranial administrations. Still other formulations include epidural, submucosal, intranasal, ocular cul-de-sac and rectal routes of administration, as well as administration by pulmonary inhalation.

If for oral administration, said composition can be a solution, a capsule, a tablet, a syrup, a paste, a gel, a quick-disintegrating tablet, a delayed-released tablet, a microdose tablet, an ointment, a cream, a powder, an aerosolized spray, or a lozenge.

If for topical administration, said composition can be a cream, an ointment, a spray, a paste, or an adhesive patch.

In a preferred embodiment, the composition of the invention enables to deliver (or contains) between 1 and 150 mg/day (for an adult) or between 10 and 50 mg/day (for a child) of amitriptyline.

According to another preferred embodiment, the composition of the invention enables to deliver (or contains) between 10 µg and 150 mg per day, preferably between 100 µg and 50 mg per day of amitriptyline.

According to another preferred embodiment, the composition of the invention enables to deliver (or contains) between 1 mg and 100 mg per day, preferably between 1 mg and 75 mg per day, more preferably between 1 mg and 50 mg per day, even more preferably between 1 mg and 15 mg per day of amitriptyline.

In a preferred embodiment, the composition of the invention is formulated so as to deliver (or contain) between 10 µg and 50 mg per day of mefloquine. More preferably, said mefloquine dose is comprised between 100 µg and 25 mg per day, even more preferably between 500 µg and 20 mg per day, even more preferably between 1 mg and 25 mg per day, and most preferably between 1 mg and 10 mg per day for an adult subject. These doses have obviously to be adjusted for the children.

In a more preferred embodiment, the composition of the invention is formulated so as to deliver between 1 mg and 100 mg per day of amitriptyline, preferably between 1 mg and 75 mg per day of amitriptyline, more preferably between 1 mg and 50 mg per day of amitriptyline, even more preferably between 1 mg and 15 mg per day of amitriptyline and between 1 mg and 25 mg per day of mefloquine per day for an adult subject, more preferably between 1 mg and 10 mg of mefloquine per day for an adult subject. This composition is for example a patch or a tablet.

Preferably, said composition is a patch or a tablet that easily deliver a determined dose of the active principles per day. Thus, the present invention targets a patch or a tablet containing the two active principles mefloquine and amitriptyline, in the daily amounts described above (e.g., between 1 mg and 50 mg of amitriptyline and between 1 mg and 25 mg of mefloquine).

The therapeutic composition of the invention is preferably used for preventing and / or treating diseases and conditions such as Major depressive disorder (MDD), post-traumatic stress disorder (PTSD), anxiety, generalized anxiety disorder (GAD), social anxiety disorder (SAD), panic disorder, neuropathic pain, resistant pain, fibromyalgia, chronic musculoskeletal pain, akynesia in Parkinson's disease, cataplexy, migraine, nocturnal enuresis, nocturnal enuresis in children, nocturnal terrors, breathing disorders, insomnia, Parkinson's disease, vasomotor symptoms of menopause, premenstrual dysphoric disorder (PMDD), restless syndrome (Willis-Ekbom disease), hypersalivation (drooling, hypersialorrhea), hypersalivation (drooling, hypersialorrhea) in amyotrophic lateral sclerosis, bipolar I disorder, bulimia, obsessive-compulsive disorder (OCD), Alzheimer's disease, Huntington's disease, multiple sclerosis, amyotrophic lateral sclerosis, Lewy body dementia, multiple system atrophy, stress, depression, depression in Parkinson disease and in dementia with Lewy bodies, schizophrenia, autism, REM sleep disorders, idiopathic REM sleep disorders, sleep bruxism, sleep disorders in fibromyalgia, sleep apnea, fibrositis

The present disclosure moreover teaches specifically the use of mefloquine and amitriptyline (preferably the hydrochloride salts thereof) in the preparation of a medicament that is intended to be used for preventing and / or treating diseases and conditions such as Major depressive disorder (MDD), post-traumatic stress disorder (PTSD), anxiety, generalized anxiety disorder (GAD), social anxiety disorder (SAD), panic disorder, neuropathic pain, resistant pain, fibromyalgia, chronic musculoskeletal pain, akynesia in Parkinson's disease, cataplexy, migraine, nocturnal enuresis, nocturnal enuresis in children, nocturnal terrors, breathing disorders, insomnia, Parkinson's disease, vasomotor symptoms of menopause, premenstrual dysphoric disorder (PMDD), restless syndrome (Willis-Ekbom disease), hypersalivation (drooling, hypersialorrhea), hypersalivation (drooling, hypersialorrhea) in amyotrophic lateral sclerosis, bipolar I disorder, bulimia, obsessive-compulsive disorder (OCD), fatigue, Alzheimer's disease, Huntington's disease, multiple sclerosis, amyotrophic lateral sclerosis, Lewy body dementia, multiple system atrophy, stress, depression, depression in Parkinson disease and in dementia with Lewy bodies, schizophrenia, autism, REM sleep disorders, idiopathic REM sleep disorders, sleep bruxism, sleep disorders in fibromyalgia, sleep apnea, fibrositis.

In a preferred embodiment, the present invention relates to the use of the composition of the invention, containing mefloquine and amitriptyline, for preventing and/or treating major depressive disorders, neuropathic pain, anxiety, fibromyalgia, vasomotor symptoms of menopause, nocturnal enuresis.

In a more preferred embodiment, the present invention relates to the use of the composition of the invention, containing mefloquine and amitriptyline, for preventing and/or treating different kinds of pain, for example post herpetic neuropathic pain, diabetic neuropathic pain, post chirurgical neuropathic pain, post chemotherapy neuropathic pain, post-stroke neuropathic pain, post cancer treatment neuropathic pain or post chemoprophylaxis neuropathic pain, post-HIV neuropathic pain, chronic back neuropathic pain, low back neuropathic pain, traumatic neuropathy, neuropathic pain related to multiple sclerosis disease or to other immune diseases, or neuropathic pain induced by spinal cord injury.

The present disclosure also teaches an *in vitro* method for identifying chemical compounds that will be efficient (and can therefore be used) for preventing and/or treating neuropathic pain, sleep disorders, or depression, said method involving testing if said compound is able to block HC, in particular, Cx43-based HC.

The tested compound will be selected if it enables to significantly block HC activity in the model which is used (15% of HC activity reduction is enough to consider that the tested compound has a potential therapeutic effect on neuropathic pain, sleep disorders, or depression).

In this method, the HC blocking activity may be detected as mentioned above, for example, by contacting said compound with activated cortical astrocytes cultures in the presence of a dye or a diffusing molecule such as EtBr.

Other characteristics of the invention will also become apparent in the course of the description which follows of the biological assays which have been performed in the framework of the invention and which provide it with the required experimental support, without limiting its scope.

### Figure legends

**Figure 1** represents HC activity studied by ethidium bromide uptake in cortical astrocyte cultures. LPS and amitriptyline were added during 24 hours to cellular medium respectively at 1µg/mL and at 5, 10 and 20 µM. Fluorescence intensity is correlated to the amount of ethidium bromide uptake. n=4-7 per condition, ***p<0.001 as compared with with LPS group, one-way ANOVA, Dunn's test.
**Figure 2** represents potentiation of amitriptyline anti-hyperalgesic effect by mefloquine, after chronic administration in SN-CCI rats. Effect of chronic administration of Amitriptyline 12 mg/kg or saline, delivered by subcutaneous osmotic pump, combined with mefloquine 0.5 mg/kg or saline administered i.p. twice a day, was evaluated on behavioural response to Randall & Selitto test in rats with unilateral chronic constriction injury to the sciatic nerve (SN-CCI). Pressure threshold values to trigger paw withdrawal (A, B) and vocalization (C, D) were determined prior to surgery (C on abscissa), then two weeks later (0 on abscissa) just before pump implantation and i.p. injection of mefloquine or saline and during the 14-day treatment. Data are the means ± S.E.M. of n=8-14 rats. Histograms (B, D) illustrate Area Under Curves, AUC (g x day) of the respective time-curves. *p<0.05, **p<0.01, ***p<0.001 as compared with "saline + saline" group at the same time, two-way ANOVA, Bonferroni test; ≠ p<0.05, ≠≠ p<0.01 as compared between amitriptyline groups, one-way ANOVA Newman-Keuls test.
**Figure 3** represents potentiation of amitriptyline anti-hyperalgesic effect by mefloquine, after repeated administration in SN-CCI rats. Amitriptyline 1; 3 mg/kg and mefloquine 1 mg/kg were administered i.p. every 150 minutes in rats with unilateral chronic constriction injury to the sciatic nerve (SN-CCI), and the paw withdrawal was evaluated on behavioural response to Randall & Selitto test. Data are the means ± S.E.M. n=9-10 *p<0.05, ***p<0.001, ****p<0.0001 as compare with control, two-way ANOVA Bonferroni post test.
**Figure 4** represents the effects of single per os administration of amitriptyline (12 and 24 mg/kg) co-administered or not with mefloquine 1 mg/kg on the swimming time in mice subjected to FST. Results obtained during the 2^{nd} 3-min periods, p<0.05 as compared with control, one-way ANOVA, Bonferroni post test.
**Figure 5** represents the inhibition of Rapid Eye Movement (REM) sleep by amitriptyline alone or combined with mefloquine. REM sleep duration was quantified in mice after treatment with amitriptyline 1 mg/kg combined or not with mefloquine 1 mg/kg. Data are the means ± S.E.M n=8-9 per group, * p<0.05 between control and amitriptyline + mefloquine groups, one-way ANOVA and Tukey post test.
**Figure 6** represents the HC activity studied by ethidium bromide uptake in cortical astrocyte cultures treated with LPS, LPS+mefloquine or LPS+MFA. LPS 1µg/mL and mefloquine or meclofenamic acid (MFA) at the dose of 10 µM were added during 24 hours to cellular medium. Fluorescence intensity is correlated to the amount of ethidium bromide uptake. n=3 per condition, ***p<0.001, as compared with LPS group ≠≠≠ p< 0.001 between MFA and mefloquine, one-way ANOVA, Dunn's test.
**Figure 7** represents the potentiation of amitriptyline anti-hyperalgesic effect by mefloquine (but not by MFA) after chronic administration in SN-CCI rats. Effect of chronic administration of Amitriptyline 12 mg/kg or saline, delivered by subcutaneous osmotic pump, combined with MFA 0.5 mg/kg (A) and mefloquine 0.5 mg/kg (B) or saline administered i.p. twice a day, was evaluated on behavioural response to Randall & Selitto test in rats with unilateral chronic constriction injury to the sciatic nerve (SN-CCI). Pressure threshold values to trigger vocalization were determined prior to surgery (C on abscissa), then two weeks later (0 on abscissa) just before pump implantation and i.p. injection of mefloquine or saline and during the 14-day treatment. Data are the means ± S.E.M. of n=8-14 rats.. *p<0.05, **p<0.01, ***p<0.001 as compared with "saline + saline" group at the same time, ≠ p<0.05 as compared between amitriptyline groups, two-way ANOVA, Bonferroni test.
**Figure 8** represents HC inhibition by amitriptyline and mefloquine. Amitriptyline 10 µM and mefloquine 0.5 µM were added to cell medium 24 hours before experiment. HeC activity studied by ethidium bromide uptake in cortical astrocyte cultures. LPS was added during 24 hours at 1 µg/mL. Fluorescence intensity is correlated to the amount of ethidium bromide uptake. n=4-6 per condition, ***p<0.001 as compared with with LPS group, one-way ANOVA, Dunn's test.

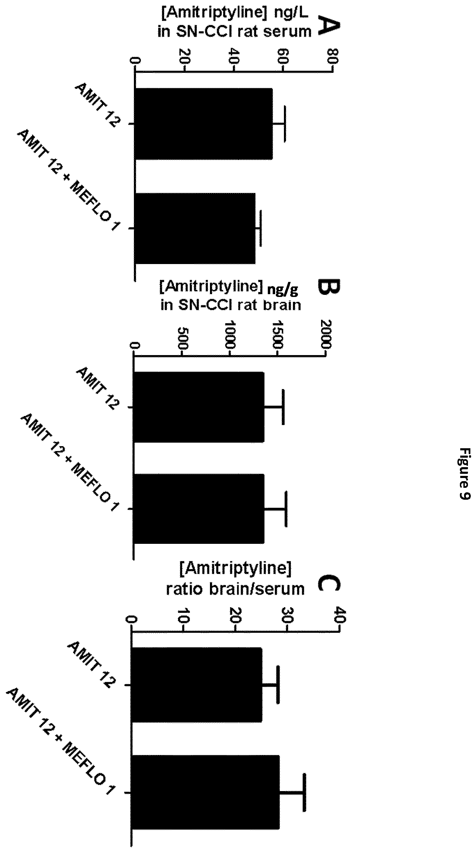

**Figure 9** represents amitriptyline pharmacokinetic in the absence or in the presence of mefloquine treatment. Amitriptyline pharmacokinetic is unchanged in serum and brain of SN-CCI rats after mefloquine treatment. Amitriptyline concentration was measured by HPLC in SN-CCI serum (A1) and brain (A2) fourteen days after chronic administration of amitriptyline (12 mg/kg, osmotic pumps) combined or not with the connexin modulator mefloquine (0,5 mg/kg i.p. bidaily). The ratio brain/plasma was also quantified (A3). n=8 per condition, no significant difference, unpaired t test.
**Figure 10** shows comparison of paw withdrawal thresholds obtained from mice undergoing von Frey tests after 25 day chronic administration of either amitriptyline 6 mg/kg alone (AMIT 6) or amitriptyline 6 mg/kg with mefloquine at 0.1 mg/kg, 0.33 mg/kg or 1 mg/kg (respectively AMIT 6 + MEFLO 0.1 (**A**) ; AMIT 6 + MEFLO 0.33 (**B**); AMIT 6 + MEFLO 1 (**C**)). Paw withdrawal thresholds of mice treated with either amitriptyline 6 mg/kg alone (AMIT 6) or amitriptyline 12 mg/kg alone (AMIT 12) are also shown (**D**). Figure 10 (**E**) represents area under curve analysis.

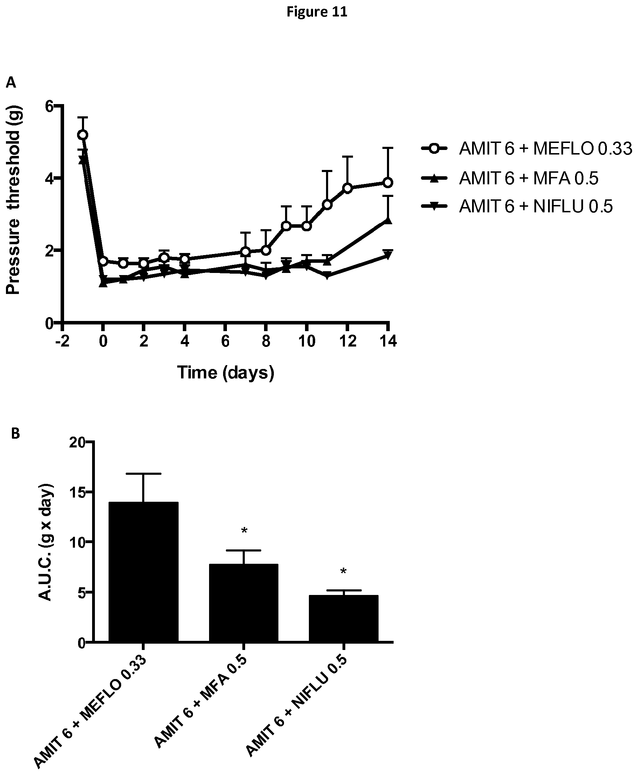

**Figure 11** represents the comparison of paw withdrawal thresholds obtained from mice undergoing von Frey tests after administration of amitriptyline 6 mg/kg with either mefloquine at 0.33 mg/kg (AMIT 6 + MEFLO 0.33), meclofenamic acid (0.5 mg/kg, MFA) or niflumic acid (0.5 mg/kg, NIFLU) (**A**). Area under curve analysis are shown (**B**).
**Figure 12** represents (**A**) Amitriptyline and (**B**) mefloquine concentrations (ng/ml) in plasma of mice treated by amitriptyline (6, 12 mg/kg) alone or combined with mefloquine (0.1, 0.3, 1 mg/kg) after 18 days of treatment. Data are presented as mean ± SEM. Kruskal-Wallis followed by Dunn's post test: **p<0.01. (**C**) Amitriptyline and (**D**) mefloquine concentration in brain (ng/g), presented here as individual values of mice treated by amitriptyline (6, 12 mg/kg) alone or combined with mefloquine (0.1, 0.3, 1 mg/kg) after 18 days of treatment. Kruskal-Wallis followed by Dunn's post test: **p<0.01.
**Figure 13** represents the coat state scores evaluated three hours after treatment in a LPS-induced model of neuro-inflammation (left graph). The right graph represents the amount of weight loss between three hours of treatment and just before treatment in a LPS-induced model of neuro-inflammation (right graph). Control mice were ip injected with saline solution. Treatments were administered orally with vehicle (CONTROL and VEH), amitriptyline 1 mg/kg combined with mefloquine 1 mg/kg (AMIT 1 + MEFLO 1) or alone (AMIT 1). In both graphs: n=4-6 mice per group, data are presented as means ±SEM and compared with a One-way ANOVA followed by Tukey's post-hoc test, *p<0.05, **p<0.05 and ***p<0.01 versus CONTROL-treated mice.

### Examples

### 1. Effects of Amitriptyline and mefloquine on neuropathic pain (hyperalgesia), depression and sleep disorders and specificity of the potentiation

### 1.1. Materiel and methods

### Cell cultures

Astrocyte cultures were prepared from cortex of rats and mice as previously described (Meme et al., 2006). Cells were seeded on polyornithine coated 35 mm dishes (5x10⁵ cells/dish) or into 100-mm diameter plastic dishes at the density of 3x10⁶ cells/dish. After 8-10 days, when cells grown into 35 mm in diameter plastic culture dishes have reached confluence, 1 µM of cytosine-arabinoside was added to the culture medium. In addition, when cells grown into 100 mm plastic culture dishes reached confluence, they were harvested with trypsin-EDTA. Then, cells were re-plated (2x10⁵ cells per well), as secondary cultures, on glass coverslips placed inside 24-round-well plate. Finally, they were grown to confluence and cytosine-arabinoside was added. Medium was changed twice a week for all cells until the experiments were carried out.

### Scrape loading/dye transfer technique

Experiments were performed on primary astrocytes grown into 35 mm plastic culture dishes. Briefly, cells were firstly incubated for 10 min, in HEPES buffered salt solution. Then cells were washed in Ca2+-free HEPES solution for 1 min and scrape loading/ dye transfer was achieved in the same Ca2+-free solution containing 1 mg/mL Lucifer yellow (LY) as previously described (Giaume et al, 1991). After 1 min, they were washed with the HEPES solution and LY loaded in the cells was allowed to diffuse through GJCs during 8 min. In all experiments, dye coupling through GJC was assessed 8 min after scraping by taking fluorescent images

### Dye Uptake in cortical astrocytes cultures

Hemichannel activity was evaluated in secondary cultures of cortical astrocytes by Ethidium Bromide (EtBr) uptake as already published *(Orellana et al, 2011).* 24h before experiment, cells were pretreated or not with 1 µg/ml LPS known to open Cx43 HeC. The day of experiment, cells were first preincubated in HEPES buffer solution. Then, cells were exposed to 5 µM EtBr for 10 min at room temperature. Cells were then washed with the same buffer before fixation with 4% paraformaldehyde in PBS. Fixed cells were examined at 40x with a confocal laser-scanning microscope. Stacks of 10 consecutive confocal images taken at 0.49 µm intervals were acquired. For each experimental condition, 6 images were captured and then analyzed with Image J program (NIH software).

### Animals

Male Sprague Dawley rats, C57BL/6 mice and Swiss CD1 male mice were respectively used for neuropathic pain, sleep-wake and FST experiments.

Animals were all accustomed to the housing facilities at their arrival and for at least 1 week before any behavioral test or surgery. Animals were maintained under the same conditions. All experiments were performed in conformity with institutional guidelines, which are in compliance with national and international laws and policies for use of animals in neuroscience research.

### Chronic constriction injury to the sciatic nerve & Behavioral testing

One week after their arrival, rats were anesthetized with sodium pentobarbital before exposing the common sciatic nerve. As described in Bennett's article, four chromic catgut (5-0) ligations were tied loosely proximally to the sciatic trifurcation (Bennett and Xie, 1988).

All behavioral assessments were conducted between 9 a.m. and 5 p.m. in a quiet room. An increasing pressure was applied to the right hind paw of SN-CCI (Sciatic Nerve-Chronic Constriction Injury) rat to evaluate hyperalgesia-like behavior (Viguier and al, 2013). Values correspond to the pressure threshold producing the paw withdrawal and then the vocalization of the rat.

### Pharmacokinetic of amitriptyline in brain and serum of rats

After a 14-day treatment with amitriptyline and mefloquine, rats were killed by decapitation. Blood was collected and kept 1 hour at 4°C. Then serum and brain were collected. Amitriptyline was quantified by HPLC and mass spectrometry (Crepta laboratory).

### Quantification of sleep-wake episodes

Mice were implanted with cortical and muscle electrodes to record the electroencephalogram (EEG) and electromyogram (EMG) and to monitor the sleep-wake cycle. This implantation allows stable and long-lasting polysomnographic recordings (Parmentier et al, 2002).

Polysomnographic records were visually scored by 10-s epochs for wakefulness (W), slow wave sleep (SWS), and paradoxical or rapid eye movement (REM) sleep.

### FST

The FST is the most used rodent model for screening antidepressants (Porsolt et al, 1977). Mice were dropped individually in a glass cylinder (25 cm high, 9 cm diameter) filled with 10 cm 25°C water. The experiment was recorded with a camcorder, placed in front of the glass cylinders. Video were scored at a later time by an experimenter blind to the treatments. The indices of depression-related behavior are the immobility time, the swimming time and the climbing time.

### Pharmacological treatments

i) For chronic neuropathic pain and pharmacokinetic experiments, amitriptyline at 12 mg/kg or 0.9% saline solution were administered to neuropathic rats, combined with mefloquine at 1 mg/kg, or vehicle (0.9% saline solution with 0.017% DMSO). Osmotic mini-pumps (Alzet, model 2ML2), delivering for the following 14 days amitriptyline or vehicle were subcutaneously implanted. Mefloquine or its vehicle were administered intraperitoneally during the 14-day treatment.
   Another protocol for neuropathic pain was performed with repeated administrations of amitriptyline 1; 3 mg/kg and mefloquine 1 mg/kg every 150 minutes.
ii) For sleep-wake experiments, mice were intraperitoneally treated at 10:30 am, before recording, with amitriptyline 1 mg/kg and mefloquine 1 mg/kg.
iii) For FST experiments, mice were orally and acutely treated by vehicle, of mefloquine (10 mg/kg), of amitriptyline at an effective dose of 24 mg/kg, and of amitriptyline at 2 ineffective doses, respectively 6 and 12 mg/kg, alone or in combination with mefloquine at 3 doses (1, 3, 10 mg/kg).

### 1.2. Results

### 1.2.1. In vitro inhibition of HC activity by amitriptyline

LPS was added to mouse cortical astrocytic cultures during 24 hours to open Cx43 hemichannels and amitriptyline was also added during the same time to evaluate its action on hemichannel activity, quantified by cell uptake of ethidium bromide.

Results are displayed on figure 1.

As expected, LPS addition to cellular medium induced the opening of hemichannel related to an increase of their activity.

Surprisingly, amitriptyline reversed this increase at the three tested doses and is able to reduce Cx43 hemichannel opening.

This result was completely unexpected, since recent publications rather show an increase of the Cx43, both in expression and function, by antidepressants. Indeed the SSRI (Selective Serotonin Reuptake inhibitor) fluoxetine and the SNRI (Serotonine Norepinephrine Reuptake Inhibitor) duloxetine have been described as increasing Cx43 expression in the prefrontal cortex after 21-day treatment in rats (Fatemi et al, 2008; Sun et al, 2012). The tricyclic antidepressant (TCA) amitriptyline has also been related to an increase of Cx43 expression as well as Cx43 gap junction channels, in mice cortical astrocytic cultures (Morioka et al, 2014), but has never been evaluated on the hemichannel activity. Consequently, it was thought so far that antidepressant administration triggers an increase of Cx43 expression and cellular coupling, whereas the results of the inventors rather show reduction of Cx functions.

### 1.2.2. Potentiation of amitriptyline activity in vivo

### 1.2.2.1. Potentiation of amitriptyline anti-hyperalgesic effect by mefloquine

Mefloquine is known to inhibit Cx36 HC, Cx30HC, and Cx43 HC (described in literature).

More precisely, mefloquine efficiently blocks hemichannels composed of several connexins, e.g., Cx26 (Levit, 2015), Cx36 (Pizarro-Delgado, 2014), Cx46 and Cx50 (Srinivas, 2005), and Cx43 (Khawaja, 2011).

The Inventors therefore hypothesized that combining the HC-blocking effect of mefloquine with the HC-blocking effect of amitriptyline would enhance the therapeutic effect of the latter.

a) Rats were ligated on sciatic nerve and then tested with the Randall & Selitto test 14 days after surgery, to evaluate hyperalgesia quantified as pressure threshold. Neuropathic rats, characterized by a decrease of pressure threshold after surgery, were then treated during 14 days and tested by the Randall & Selitto test during this period.

Results are disclosed on figure 2.

Chronic administration of amitriptyline 12 mg/kg induced a significant increase of the paw withdrawal threshold at day 14 compared to saline as well as an increase of the vocalization threshold.

Mefloquine did not present significant effect on both responses but significantly potentiated amitriptyline hyperalgesic profile from day 6 to day 14.

Area under curve analysis confirmed this potentiation (fig 2 B). Similar results were obtained by the quantification of vocalization threshold (fig. 2C and D).

b) After ligation of the sciatic nerve, rats were treated by amitriptyline and mefloquine each 150 minutes (amitriptyline half-life) and then tested at 30, 90, 240 and 390 minutes with the Randall & Selitto test.

Results are disclosed on figure 3.

Significant effects of amitriptyline were observed at 390 min for paw withdrawal at the dose of 1 mg/kg in the Randall & Selitto test.

In addition, mefloquine significantly improved the anti-hyperalgesic effect of amitriptyline, indeed significant and higher values were obtained at 90 and 240 min whereas amitriptyline did not induce significant effect on its own.

### 1.2.2.2. Potentiation of anti-depressant effect of amitriptyline by mefloquine

Mice were subjected to FST, after administration of amitriptyline and mefloquine. FST test evaluates the despair behavior which is commonly reversed by antidepressants. During FST, the swimming time of each mouse was measured on each 1-min period, on 1^{st} and 2^{nd} 3-min periods.

Results are disclosed on figure 4.

During the 2^{nd} 3-min periods, a dose effect of amitriptyline was obtained with significant increase of the swimming time at the highest dose (24 mg/kg).

In addition, mefloquine 1 mg/kg was able to potentiate amitriptyline 12 mg/kg.

Interestingly, the combination of both drugs at doses 12 mg/kg was significant only in presence of mefloquine 1 mg/kg (the effect of amitriptyline 12 mg/kg alone was not significant).

### 1.2.2.3. Potentiation of amitriptyline by mefloquine on REM sleep

Mice were registered by electroencephalogram to quantify sleep-wake episodes. Then, following acute administrations of amitriptyline and mefloquine, different polysomnographic records, including Rapid Eye Movement (REM) sleep, were quantified.

Results are disclosed on figure 5.

REM sleep was significantly reduced by amitriptyline + mefloquine in mice, whereas amitriptyline did not induce significant reduction on its own.

Therefore this result is in favor of a potentiation by mefloquine of the inhibitor effect of amitriptyline on REM sleep.

Altogether, these results obtained in different relevant preclinical models related to clinical applications of amitriptyline (neuropathic pain, depression) or markers of its activity (REM sleep reduction), clearly show that mefloquine acts synergistically *in vivo* for potentiating the antidepressant effects of amitriptyline. Importantly, the mefloquine doses used to potentiate amitriptyline effects are very low (mefloquine has therefore no effect on its own).

### 1.2.3. Specificity of amitriptyline potentiation by mefloquine

1.2.3.1. *MFA is a potent HC blocking agent.*

Meclofenamic acid (MFA) is referred to as a hemichannel blocker (Richter, 2014).

To verify this, LPS was added to mouse cortical astrocytic cultures during 24 hours to open Cx43 hemichannels and mefloquine or meclofenamic acid (MFA) were also added during the same time to evaluate their action on hemichannel activity, quantified by cell uptake of ethidium bromide.

Results are disclosed on figure 6.

As expected, LPS addition to cellular medium induced the opening of hemichannel related to an increase of their activity. MFA and mefloquine both reversed this effect with a significant difference between their own effects, but MFA presented a higher inhibitor effect.

Therefore, MFA and mefloquine are able to reduce Cx43 hemichannel opening with different inhibition degrees, mefloquine being less efficient in this respect.

1.2.3.2. *Yet, MFA does not enhance amitriptyline effect in vivo.*

Rats were ligated on sciatic nerve and then tested with the Randall & Selitto test 14 days after surgery, to evaluate hyperalgesia quantified as pressure threshold. Neuropathic rats, characterized by a decrease of pressure threshold after surgery, were then treated during 14 days and tested by the Randall & Selitto test during this period.

Results are disclosed on figure 7.

Chronic administration of amitriptyline 12 mg/kg induced a significant increase of the paw withdrawal threshold at day 14 compared to saline as well as an increase of the vocalization threshold. MFA did not present any improved effect of amitriptyline whereas mefloquine significantly potentiated amitriptyline hyperalgesic at day 14.

### 1.2.3.3. No synergistic HC activity in the presence of amitriptyline combined with mefloquine

After 24-hour treatment of rat cortical astrocytic cultures, with amitriptyline and/or mefloquine, Cx43 hemichannel activity was evaluated by uptake of EtBr.

Results are displayed on figure 8.

Significant inhibition effects of both treatments on hemichannel activity were identified by evaluation of ethidium bromide uptake in cultured cortical rat astrocytes. Around 30% of reduction was observed in each treated group after 24-hour treatment, but without any synergistic effect.

The synergistic effect of amitriptyline by mefloquine observed *in vivo* does not seem uniquely related to improved hemichannel inhibition given that combination of both treatments did not induce higher inhibitor effect than amitriptyline alone. Moreover, increased inhibition of HC activity by MFA does not lead to potentiation *in vivo* when combined with amitriptyline. In addition these results reinforce the specificity of mefloquine to improve amitriptyline anti-hyperalgesic effect.

### 1.2.4. Amitriptyline pharmacokinetic in presence of mefloquine

Rats were ligated on sciatic nerve and treated by amitriptyline, combined or not with mefloquine, 14 days after surgery. After 14-day treatment, so 28 days after surgery, rats were sacrificed and brain as well as serum samples were collected to quantify amitriptyline.

Results are displayed on figure 9.

No changes of amitriptyline concentration were observed after mefloquine 14-day treatment neither for serum nor for brain pharmacokinetic.

MFA, mefloquine, and more surprisingly amitriptyline, inhibit connexin hemichannels. More importantly, MFA, while inhibiting those hemichannels better than mefloquine, does not potentiate amitriptyline anti-hyperalgesic profile, while mefloquine significantly does. This last potentiation is not due to pharmacokinetic modification of amitriptyline either in blood or in brain.

### 2. Effects of Amitriptyline and mefloquine on allodynia ("Cuff model")

### 2.1. Materiel and methods

### Surgeries

Neuropathic pain was induced to adult male C57BL/6J mice (Charles River, L'Arbresle, France) by cuffing the main branch of the right sciatic nerve (For detailed description and video illustration: Yalcin et al. 2014, J Vis Exp 89:e51608). Surgeries were performed under ketamine (68 mg/kg) / xylazine (10 mg/kg) intraperitoneal (i.p.) anesthesia (Centravet, Tadden, France). The common branch of the right sciatic nerve was exposed and a cuff of PE-20 polyethylene tubing (Harvard Apparatus, Les Ulis, France) of standardized length (2 mm) was unilaterally inserted around it (Cuff group). The shaved skin was closed using suture.

### Animal treatments

Mice received i.p. injections of amitriptyline (6, 12 mg/kg) and mefloquine (0.1, 0.33, 1 mg/kg), in a volume of 10 mL/kg during 25 days. Injections were done once a day during the 17 first days of treatment, in the afternoon. As a single dose per day was ineffective, treatment was then done twice a day, morning and afternoon, from the 8 last days of treatment.

### Evaluation of mechanical allodynia

Mechanical allodynia was tested using von Frey hairs and results were expressed in grams (For detailed description and video illustration: Yalcin et al. 2014, J Vis Exp 89:e51608). Tests were done during the morning, starting at least 2 hours after lights on. Mice were placed in clear Plexiglas boxes (7 cm x 9 cm x 7 cm) on an elevated mesh screen. Calibrated von Frey filaments (Bioseb, Vitrolles, France) were applied to the plantar surface of each hindpaw until they just bent, in a series of ascending forces up to the mechanical threshold. Filaments were tested five times per paw and the paw withdrawal threshold was defined as the lower of two consecutive filaments for which three or more withdrawals out of the five trials were observed.

### Comparison of the effect of different combinations on mechanical allodynia

Mice were subjected to sciatic nerve cuffing and then tested with the von Frey test to evaluate allodynia quantified as pressure threshold. Neuropathic mice, characterized by a decrease of pressure threshold after surgery, were then treated during 14 days with amitriptyline 6 mg/kg associated with mefloquine (0.33 mg/kg, MEFLO), meclofenamic acid (0.5 mg/kg, MFA) and niflumic acid (0.5 mg/kg, NIFLU) and tested by the von Frey test during this period.

### 2.2. Results

### 2.2.1. Potentiation of amitriptyline activity on allodynia ("Cuff model")

Mice were subjected to sciatic nerve cuffing and then tested with the von Frey test to evaluate allodynia quantified as pressure threshold. Neuropathic mice, characterized by a decrease of pressure threshold after surgery, were then treated during 25 days and tested by the von Frey test during this period.

Results are disclosed on figure 10.

Chronic administration of amitriptyline 12 mg/kg induced a significant increase of the paw withdrawal threshold from day 32 to day 34, in comparison with amitriptyline 6 mg/kg. At this lower dose, amitriptyline induced no changes of paw withdrawal in neuropathic mice.

Mefloquine at the three tested doses significantly potentiated amitriptyline 6 mg/kg. Moreover, the lowest doses of mefloquine (0.1 and 0.33 mg/kg) induced antiallodynic effect similar to amitriptyline 12 mg/kg whereas the highest dose (1 mg/kg) presented lower efficiency to potentiate amitriptyline.

Area under curve analysis confirmed this potentiation.

These results show potentiation of amitriptyline beneficial effects by mefloquine in neuropathic pain disease, and in particular in one of the specific symptoms of neuropathic pain (allodynia).

### 2.2.2. Specificity of amitriptyline potentiation by mefloquine

Figure 11 shows that the combination of amitriptyline with mefloquine is significantly more efficient than the combinations of amitriptyline with other connexin inhibitors such as meclofenamic acid and niflumic acid (Retamal et al., 2007), as allodynia is reversed more rapidly for this specific combination.

This more potent synergistic effect of amitriptyline combined with mefloquine over combinations of amitriptyline with meclofenamic acid and niflumic acid is further confirmed through AUC quantification (One-Way Anova followed by Holm-Sidak multiple comparison test).

### 3. Pharmacokinetics of amitriptyline / mefloquine in mice

### 3.1. Material and methods

### Animals

Adult male C57BL/6J mice (Charles River, L'Arbresle, France) were used. Mice were kept in collective cages for habituation to the facility for one week before experimental testing with food and water *ad libitum.* Mice were maintained on a light/dark cycle of 12 hours with light onset at 8h00 and with a controlled temperature of 21°C ± 1°C.

### Treatments

Mice received intraperitoneal injections of amitriptyline (6, 12 mg/kg) and mefloquine (0.1, 0.3 and 1 mg/kg), in a volume of 10 mL/kg during 18 days. Injections were done twice.

### Blood and brain collection

On day 18^{th} of treatment, mice received the morning injection and samplings were done 2 hours after the injection. Mice were anesthetized just before sampling (ketamine 68 mg/kg, xylazine 10 mg/kg) and blood was collected by intracardiac puncture (Vacuette tubes, with lithium/heparin gel). The mouse was then decapitated, and the brain extracted and fast frozen in dry ice.

Vacuette tubes with blood were centrifugated (3000 rpm, 20 min), plasma recovered and stored at -80°C until shipping in dry ice.

### Amitriptyline and mefloquine determination

In brain and serum, amitriptyline and mefloquine were both quantified by Liquid Chromatography and Mass Spectrometry (LC-MS) method.

### 3.2. Results

Amitriptyline and mefloquine were quantified in plasma and brain after 18 days of treatments, in mice previously tested for allodynia after sciatic nerve cuffing.

Figure 12 shows that mefloquine and amitriptyline concentrations are dose-dependent in blood and brain tissue.

In addition, Figure 12 shows that mefloquine does not modulate amitriptyline pharmacokinetics profile when both compounds are used at doses allowing the potentiation effect (i.e. 6 and 12 mg/kg amitriptyline and 0.1, 0.3 and 1 mg/kg mefloquine). Importantly, mefloquine does not induce an increase the concentrations of amitriptyline in blood and brain, even for the highest doses of mefloquine.

Interestingly, these results show that optimal potentiation doses lead to a plasma exposure equivalent to about 10% of plasma exposure obtained with the doses of mefloquine that are conventionally used (usually 1000 to 2000 ng/mL). Higher doses of mefloquine, ie closer to those conventionally used, are less effective for potentiating amitriptyline. Here, the in vivo potentiation is optimal at concentrations of the micromolar scale.

For example, Margineanu and Klitgaard, 2006, describes an *in vitro* inhibitory effect of connexins with a mefloquine brain concentration between 100 and 200 µM. In contrast, the inventors demonstrated that the *in vivo* potentiation of amitriptyline is optimal with mefloquine concentrations in plasma and brain of the micromolar scale, that is 100x less (figures 12).

### 4. Effects of amitriptyline / mefloquine on neuro-inflammation (LPS model)

### 4.1. Material and methods

### Animals

Adult Swiss male mice were used in the LPS model. Mice were kept in collective cages for habituation to the facility for one week before experimental testing. Afterwards, they were housed in individual cages two days before the experiment started with food and water *ad libitum.* Mice were maintained on a light/dark cycle of 12 hours with light onset at 8h00 and with a controlled temperature of 21°C ± 1°C.

Experimenter was blind to treatment.

### LPS model

After two days of isolation in individual cages, mice were injected intraperitoneally with either lipopolysaccharide (LPS; O127:B7, Sigma-Aldrich) at 0.33 mg/kg or saline solution. Twenty-one hours after first injection, the mice received their treatment orally.

### Grooming activity

Coat state score was evaluated three hours after treatment. The score was defined as the sum of five notes (0 or 1) corresponding to the equal number of graded body parts (head, neck, dorsal coat, ventral coat and, hindlegs and genital region). The cleanness fur state was evaluated for each of these five body parts. The note of 0 was attributed to white and clean fur and the note of 1 was attributed to altered fur (spikes, yellow-ish colour, greasy fur).

### Weight loss

General physiological state of the animals was assessed by the normalised weight loss; defined as the difference between weight measures three hours after treatment (T0+24h) and just before treatment (T0+21h).

### 4.1. Results

Inflammation has been described as a component of the pathogenesis of several neurodegenerative diseases **(Alzheimer's disease, Parkinson's disease, Huntington's disease, multiple sclerosis and amyotrophic lateral sclerosis).** It implicates the activation of brain microglia and release of pro-inflammatory cytokines such as IL-1β and TNFα, which participate in neuro-inflammation and neurotoxicity. (Qin et al., 2010).

It has been demonstrated that peripheral administration of LPS triggers a release of pro-inflammatory cytokines, hence mimicking the pathophysiology of neurodegenerative disorders. Moreover, it has been recently reported that the neuro-inflammation induced by LPS can also mimick **psychiatric disorders symptoms/features in humans and in animal model such as stress, depression, bipolar disorders, schizophrenia and autism.** Therefore, LPS model has been widely used to characterize new molecules in the neuro-inflammation and might be an interesting model to screen new drugs targeting neurodegenerative and psychiatric disorders. (Réus et al., 2015).

Coat state score allows to evaluate grooming activity. This activity has been described to reflect the stress underwent by mice. Indeed, a stressed mouse will not take time to groom and stay clean. Therefore, the general coat state score will be elevated. The left panel of figure 13 represents the mean of animal coat state score. LPS induces a significant increase in scores (p<0.01) compared to control (saline + vehicle) mice. Amitriptyline treatment has a tendency to reduce the deterioration of coat state. But when amitriptyline and mefloquine are co-administrated, the reduction in coat state deterioration markedly increased.

Weight measure allows to assess the general state of sickness induced by neuro-inflammation. The right panel of figure 13 shows that LPS-administrated mice have a significantly lower weight than control mice. Amitriptyline treatment induces a decrease in weight loss. Treatment with the amitriptyline and mefloquine combination induces a significantly greater decrease in weight loss.

These results show potentiation of amitriptyline beneficial effects by mefloquine in neuro-inflammation.

### BIBLIOGRAPHIC REFERENCES

Avshalumova, L., Fabrikant, J., & Koriakos, A. (2014). Overview of skin diseases linked to connexin gene mutations. IntJ Dermatol, 53(2), 192-205. doi: 10.1111/ijd.12062
Babu, N. A., Rajesh, E., Krupaa, J., & Gnananandar, G. (2015). Genodermatoses. J Pharm Bioallied Sci, 7(Suppl 1), S203-206. doi: 10.4103/0975-7406.155903
Baroja-Mazo, A., Barbera-Cremades, M., & Pelegrin, P. (2013). The participation of plasma membrane hemichannels to purinergic signaling. Biochim Biophys Acta, 1828(1), 79-93. doi: 10.1016/j.bbamem.2012.01.002
Behbehani, R. (2007). Clinical approach to optic neuropathies. Clin Ophthalmol, 1(3), 233-246.
Bermudez, L. E., Inderlied, C. B., Kolonoski, P., Chee, C. B., Aralar, P., Petrofsky, M., ... Young, L. S. (2012). Identification of (+)-erythro-mefloquine as an active enantiomer with greater efficacy than mefloquine against Mycobacterium avium infection in mice. Antimicrob Agents Chemother, 56(8), 4202-4206. doi: 10.1128/AAC.00320-12
Beyer, E. C., & Berthoud, V. M. (2014). Connexin hemichannels in the lens. Front Physiol, 5, 20. doi: 10.3389/fphys.2014.00020
Burkovetskaya, M., Karpuk, N., Xiong, J., Bosch, M., Boska, M. D., Takeuchi, H., ... Kielian, T. (2014). Evidence for aberrant astrocyte hemichannel activity in Juvenile Neuronal Ceroid Lipofuscinosis (JNCL). PLoS One, 9(4), e95023. doi: 10.1371/journal.pone.0095023
Campbell, J. N., & Meyer, R. A. (2006). Mechanisms of neuropathic pain. Neuron, 52(1), 77-92. doi: 10.1016/j.neuron.2006.09.021
Chen, M. J., Kress, B., Han, X., Moll, K., Peng, W., Ji, R. R., & Nedergaard, M. (2012). Astrocytic CX43 hemichannels and gap junctions play a crucial role in development of chronic neuropathic pain following spinal cord injury. Glia, 60(11), 1660-1670. doi: 10.1002/glia.22384
Chen, Y. S., Toth, I., Danesh-Meyer, H. V., Green, C. R., & Rupenthal, I. D. (2013). Cytotoxicity and vitreous stability of chemically modified connexin43 mimetic peptides for the treatment of optic neuropathy. J Pharm Sci, 102(7), 2322-2331. doi: 10.1002/jps.23617
Danesh-Meyer, H. V., Kerr, N. M., Zhang, J., Eady, E. K., O'Carroll, S. J., Nicholson, L. F., ... Green, C. R. (2012). Connexin43 mimetic peptide reduces vascular leak and retinal ganglion cell death following retinal ischaemia. Brain, 135(Pt 2), 506-520. doi: 10.1093/brain/awr338
Davidson, J. O., Green, C. R., Nicholson, L. F., Bennet, L., & Gunn, A. J. (2013). Connexin hemichannel blockade is neuroprotective after, but not during, global cerebral ischemia in near-term fetal sheep. Exp Neurol. doi: 10.1016/j.expneurol.2013.06.026
Davidson, J. O., Green, C. R., Nicholson, L. F., O'Carroll, S. J., Fraser, M., Bennet, L., & Gunn, A. J. (2012). Connexin hemichannel blockade improves outcomes in a model of fetal ischemia. Ann Neurol, 71(1), 121-132. doi: 10.1002/ana.22654
De Bock, M., Kerrebrouck, M., Wang, N., & Leybaert, L. (2013). Neurological manifestations of oculodentodigital dysplasia: a Cx43 channelopathy of the central nervous system? Front Pharmacol, 4, 120. doi: 10.3389/fphar.2013.00120
Diekmann, S., Henneke, M., Burckhardt, B. C., & Gartner, J. (2010). Pelizaeus-Merzbacher-like disease is caused not only by a loss of connexin47 function but also by a hemichannel dysfunction. Eur J Hum Genet, 18(9), 985-992. doi: 10.1038/ejhg.2010.61
Durham, P. L., & Garrett, F. G. (2009). Neurological mechanisms of migraine: potential of the gap-junction modulator tonabersat in prevention of migraine. Cephalalgia, 29 Suppl 2, 1-6. doi: 10.1111/j.1468-2982.2009.01976.x
EIBatsh, M. M. (2015). Antidepressant-like effect of simvastatin in diabetic rats. Can J Physiol Pharmacol, 1-8. doi: 10.1139/cjpp-2014-0560
Essenfelder, G. M., Bruzzone, R., Lamartine, J., Charollais, A., Blanchet-Bardon, C., Barbe, M. T., Waksman, G. (2004). Connexin30 mutations responsible for hidrotic ectodermal dysplasia cause abnormal hemichannel activity. Hum Mol Genet, 13(16), 1703-1714. doi: 10.1093/hmg/ddh191
Fatemi, S. H., Folsom, T. D., Reutiman, T. J., Pandian, T., Braun, N. N., & Haug, K. (2008). Chronic psychotropic drug treatment causes differential expression of connexin 43 and GFAP in frontal cortex of rats. Schizophr Res, 104(1-3), 127-134.
Ferini-Strambi, L., Marelli, S., Galbiati, A., Rinaldi, F., & Giora, E. (2014). REM Sleep Behavior Disorder (RBD) as a marker of neurodegenerative disorders. Arch Ital Biol, 152(2-3), 129-146. doi: 10.12871/000298292014238
Ferman, T. J., Boeve, B. F., Smith, G. E., Silber, M. H., Kokmen, E., Petersen, R. C., & Ivnik, R. J. (1999). REM sleep behavior disorder and dementia: cognitive differences when compared with AD. Neurology, 52(5), 951-957.
Finnerup, N. B., Sindrup, S. H., & Jensen, T. S. (2010). The evidence for pharmacological treatment of neuropathic pain. Pain, 150(3), 573-581. doi: 10.1016/j.pain.2010.06.019
Fontaine, F., de Sousa, G., Burcham, P. C., Duchene, P., & Rahmani, R. (2000). Role of cytochrome P450 3A in the metabolism of mefloquine in human and animal hepatocytes. Life Sci, 66(22), 2193-2212.
Gerner, P., Haderer, A. E., Mujtaba, M., Sudoh, Y., Narang, S., Abdi, S., ... Wang, G. K. (2003). Assessment of differential blockade by amitriptyline and its N-methyl derivative in different species by different routes. Anesthesiology, 98(6), 1484-1490.
Giaume, C., Leybaert, L., C, C. Naus, & J, C. Saez. (2013). Connexin and pannexin hemichannels in brain glial cells: properties, pharmacology, and roles. Front Pharmacol, 4, 88. doi: 10.3389/fphar.2013.00088
Giaume, C., Leybaert, L., Naus, C. C., & Saez, J. C. (2013). Connexin and pannexin hemichannels in brain glial cells: properties, pharmacology, and roles. Front Pharmacol, 4, 88. doi: 10.3389/fphar.2013.00088
Giaume, C., & Theis, M. (2010). Pharmacological and genetic approaches to study connexin-mediated channels in glial cells of the central nervous system. Brain Res Rev, 63(1-2), 160-176. doi: 10.1016/j.brainresrev.2009.11.005
Guo, K., Zhu, J., Quan, X., Zhang, D., Chen, X., Yang, C., ... Zhu, Q. (2015). Comparison of the effects of pretreatment with repeated electroacupuncture at GV20 and ST36 on fatigue in rats. Acupunct Med. doi: 10.1136/acupmed-2014-010686
Hobson, G. M., & Garbern, J. Y. (2012). Pelizaeus-Merzbacher disease, Pelizaeus-Merzbacher-like disease 1, and related hypomyelinating disorders. Semin Neurol, 32(1), 62-67. doi: 10.1055/s-0032-1306388
Huang, Y., Huang, P., Ren, X., & Zhang, J. (2015). [Effects of CX43 hemichannel on neuronal damage after ischemia-reperfusion injury]. Zhonghua Yi Xue Za Zhi, 95(3), 221-225.
Izzo, V., Pagnoni, B., & Rigoli, M. (1991). Recent acquisitions in pain therapy: meclofenamic acid. Clin J Pain, 7 Suppl 1, S49-53.
Jan, A. Y., Amin, S., Ratajczak, P., Richard, G., & Sybert, V. P. (2004). Genetic heterogeneity of KID syndrome: identification of a Cx30 gene (GJB6) mutation in a patient with KID syndrome and congenital atrichia. J Invest Dermatol, 122(5), 1108-1113. doi: 10.1111/j.0022-202X.2004.22518.x
Jeanson T., Figueiredo A.C., Bourgoin S., Picoli C., Mouthon F., Giaume C., Charvériat M, Hamon M. Connexin channel inhibitor promotes the anti-hyperalgesic effect of amitriptyline in sciatic nerve-ligated rats. European neuropsychopharmacology. October 2014
Juszczak, G. R., & Swiergiel, A. H. (2009). Properties of gap junction blockers and their behavioural, cognitive and electrophysiological effects: animal and human studies. Prog Neuropsychopharmacol Biol Psychiatry, 33(2), 181-198. doi: 10.1016/j.pnpbp.2008.12.014
Khawaja, Kiran. (2011). Involvement of connexin 43 in ATP release from endothelial cells during reoxygenation. Role of PKA signaling pathway. Inaugural Dissertation*.*
Koulakoff, A., Mei, X., Orellana, J. A., Saez, J. C., & Giaume, C. (2012). Glial connexin expression and function in the context of Alzheimer's disease. Biochim Biophys Acta, 1818(8), 2048-2057. doi: 10.1016/j.bbamem.2011.10.001
Lee, K. M., Coehlo, M., McGregor, H. A., Waltermire, R. S., & Szumlinski, K. K. (2015). Binge alcohol drinking elicits persistent negative affect in mice. Behav Brain Res, 291, 385-398. doi: 10.1016/j.bbr.2015.05.055
Levit, N. A., Sellitto, C., Wang, H. Z., Li, L., Srinivas, M., Brink, P. R., & White, T. W. (2015). Aberrant connexin26 hemichannels underlying keratitis-ichthyosis-deafness syndrome are potently inhibited by mefloquine. J Invest Dermatol, 135(4), 1033-1042. doi: 10.1038/jid.2014.408
Maes, M., Crespo Yanguas, S., Willebrords, J., Cogliati, B., & Vinken, M. (2015). Connexin and pannexin signaling in gastrointestinal and liver disease. Transl Res. doi: 10.1016/j.trsl.2015.05.005
Maj, J., Vetulani, J., Michaluk, J., Rogoz, Z., & Skuza, G. (1982). Central action of amitriptyline N-oxide. Pharmacopsychiatria, 15(6), 187-191. doi: 10.1055/s-2007-1019536
Mandal, A., Shahidullah, M., & Delamere, N. A. (2015). Calcium entry via connexin hemichannels in lens epithelium. Exp Eye Res, 132, 52-58. doi: 10.1016/j.exer.2015.01.012
Margineanu, DG. and Klitgaard, H. (2006). The connexin 36 blockers quinine, quinidine and mefloquine inhibit cortical spreading depression in a rat neocortical slice model in vitro. Brain Res. Bulletin, 71, 23-26.
Moore, R. A., Derry, S., Aldington, D., Cole, P., & Wiffen, P. J. (2015). Amitriptyline for neuropathic pain in adults. Cochrane Database Syst Rev, 7, CD008242. doi: 10.1002/14651858.CD008242.pub3
Morioka, N., Suekama, K., Zhang, F. F., Kajitani, N., Hisaoka-Nakashima, K., Takebayashi, M., & Nakata, Y. (2014). Amitriptyline up-regulates connexin43-gap junction in rat cultured cortical astrocytes via activation of the p38 and c-Fos/AP-1 signalling pathway. Br J Pharmacol, 171(11), 2854-2867. doi: 10.1111/bph.12614
Mouri, A., Noda, Y., Enomoto, T., & Nabeshima, T. (2007). Phencyclidine animal models of schizophrenia: approaches from abnormality of glutamatergic neurotransmission and neurodevelopment. Neurochem Int, 51(2-4), 173-184. doi: 10.1016/j.neuint.2007.06.019
Muller, M., Orben, C. M., Schutzenmeister, N., Schmidt, M., Leonov, A., Reinscheid, U. M., ... Griesinger, C. (2013). The absolute configuration of (+)- and (-)-erythro-mefloquine. Angew Chem Int Ed Engl, 52(23), 6047-6049. doi: 10.1002/anie.201300258
Musa, M. N. (1988). Sleep apnea following withdrawal of amitriptyline. J Clin Pharmacol, 28(11), 1038-1039.
Nabeshima, T., Mouri, A., Murai, R., & Noda, Y. (2006). Animal model of schizophrenia: dysfunction of NMDA receptor-signaling in mice following withdrawal from repeated administration of phencyclidine. Ann N YAcad Sci, 1086, 160-168. doi: 10.1196/annals.1377.003
Nakase, T., & Naus, C. C. (2004). Gap junctions and neurological disorders of the central nervous system. Biochim Biophys Acta, 1662(1-2), 149-158.
O'Carroll, S. J., Gorrie, C. A., Velamoor, S., Green, C. R., & Nicholson, L. F. (2013). Connexin43 mimetic peptide is neuroprotective and improves function following spinal cord injury. Neurosci Res, 75(3), 256-267. doi: 10.1016/j.neures.2013.01.004
Ohara, P. T., Vit, J. P., Bhargava, A., & Jasmin, L. (2008). Evidence for a role of connexin 43 in trigeminal pain using RNA interference in vivo. J Neurophysiol, 100(6), 3064-3073. doi: 10.1152/jn.90722.2008
Orellana, J. A., Hernandez, D. E., Ezan, P., Velarde, V., Bennett, M. V., Giaume, C., & Saez, J. C. (2010). Hypoxia in high glucose followed by reoxygenation in normal glucose reduces the viability of cortical astrocytes through increased permeability of connexin 43 hemichannels. Glia, 58(3), 329-343. doi: 10.1002/glia.20926
Orellana, J. A., Martinez, A. D., & Retamal, M. A. (2013). Gap junction channels and hemichannels in the CNS: regulation by signaling molecules. Neuropharmacology, 75, 567-582. doi: 10.1016/j.neuropharm.2013.02.020
Palagini, L., Baglioni, C., Ciapparelli, A., Gemignani, A., & Riemann, D. (2013). REM sleep dysregulation in depression: state of the art. Sleep Med Rev, 17(5), 377-390. doi: 10.1016/j.smrv.2012.11.001
Pasero, C. (2004). Pathophysiology of neuropathic pain. Pain Manag Nurs, 5(4 Suppl 1), 3-8. doi: 10.1016/j.pmn.2004.10.002
Patel, S. J., Milwid, J. M., King, K. R., Bohr, S., Iracheta-Velle, A., Li, M., ... Yarmush, M. L. (2012). Gap junction inhibition prevents drug-induced liver toxicity and fulminant hepatic failure. Nat Biotechnol, 30(2), 179-183. doi: 10.1038/nbt.2089
Paznekas, W. A., Karczeski, B., Vermeer, S., Lowry, R. B., Delatycki, M., Laurence, F., ... Jabs, E. W. (2009). GJA1 mutations, variants, and connexin 43 dysfunction as it relates to the oculodentodigital dysplasia phenotype. Hum Mutat, 30(5), 724-733. doi: 10.1002/humu.20958
Pizarro-Delgado, J., Fasciani, I., Temperan, A., Romero, M., Gonzalez-Nieto, D., Alonso-Magdalena, P., ... Tamarit-Rodriguez, J. (2014). Inhibition of connexin 36 hemichannels by glucose contributes to the stimulation of insulin secretion. Am J Physiol Endocrinol Metab, 306(12), E1354-1366. doi: 10.1152/ajpendo.00358.2013
Postuma, R. B., Iranzo, A., Hogl, B., Arnulf, I., Ferini-Strambi, L., Manni, R., Montplaisir, J. Y. (2015). Risk factors for neurodegeneration in idiopathic rapid eye movement sleep behavior disorder: a multicenter study. Ann Neurol, 77(5), 830-839. doi: 10.1002/ana.24385
Qin L, Liu Y, , Crews FT. (2013) NADPH oxidase and aging drive microglial activation, oxidative stress, and dopaminergic neurodegeneration following systemic LPS administration Glia. Jun;61(6):855-68. doi: 10.1002/glia.22479
Rafael, Linden, Venzon, Antunes Marina, Luiza, Ziulkoski Ana, Maina, Wingert, Paula, Tonello, Mladen, Tzvetkov, & Arigony, Souto Andre. (2008). Determination of amitriptyline and its main metabolites in human plasma samples using HPLC-DAD: application to the determination of metabolic ratios after single oral dose of amitriptyline. Journal of the Brazilian Chemical Society. doi: 10.1590/s0103-50532008000100007
Retamal MA, Froger N, Palacios-Prado N, Ezan P, Sáez PJ, Sáez JC, Giaume C.J Neurosci. 2007 Dec 12;27(50):13781-92. Cx43 hemichannels and gap junction channels in astrocytes are regulated oppositely by proinflammatory cytokines released from activated microglia.
Réus GZ, Fries GR, Stertz L, Badawy M, Passos IC, Barichello T, Kapczinski F, Quevedo J. (2015). The role of inflammation and microglial activation in the pathophysiology of psychiatric disorders. Neuroscience. Aug 6;300:141-54. doi: 10.1016/j.neuroscience.05.018
Rhodes, J. D., & Sanderson, J. (2009). The mechanisms of calcium homeostasis and signalling in the lens. Exp Eye Res, 88(2), 226-234. doi: 10.1016/j.exer.2008.10.025
Richter, K., Kiefer, K. P., Grzesik, B. A., Clauss, W. G., & Fronius, M. (2014). Hydrostatic pressure activates ATP-sensitive K+ channels in lung epithelium by ATP release through pannexin and connexin hemichannels. FASEB J, 28(1), 45-55. doi: 10.1096/fj.13-229252
Roizenblatt, S., Neto, N. S., & Tufik, S. (2011). Sleep disorders and fibromyalgia. Curr Pain Headache Rep, 15(5), 347-357. doi: 10.1007/s11916-011-0213-3
Russo, E., Citraro, R., Davoli, A., Gallelli, L., Di Paola, E. D., & De Sarro, G. (2013). Ameliorating effects of aripiprazole on cognitive functions and depressive-like behavior in a genetic rat model of absence epilepsy and mild-depression comorbidity. Neuropharmacology, 64, 371-379. doi: 10.1016/j.neuropharm.2012.06.039
Sahin Onat, S., & Malas, F. U. (2014). Duloxetine-induced Sleep Bruxism in Fibromyalgia Successfully Treated With Amitriptyline. Acta Reumatol Port.
Sanchez, H. A., & Verselis, V. K. (2014). Aberrant Cx26 hemichannels and keratitis-ichthyosis-deafness syndrome: insights into syndromic hearing loss. Front Cell Neurosci, 8, 354. doi: 10.3389/fncel.2014.00354
Srinivas, M., Kronengold, J., Bukauskas, F. F., Bargiello, T. A., & Verselis, V. K. (2005). Correlative studies of gating in Cx46 and Cx50 hemichannels and gap junction channels. Biophys J, 88(3), 1725-1739. doi: 10.1529/biophysj.104.054023
Staner, L. (2010). Comorbidity of insomnia and depression. Sleep Med Rev, 14(1), 35-46. doi: 10.1016/j.smrv.2009.09.003
Sun, J. D., Liu, Y., Yuan, Y. H., Li, J., & Chen, N. H. (2012). Gap junction dysfunction in the prefrontal cortex induces depressive-like behaviors in rats. Neuropsychopharmacology, 37(5), 1305-1320. doi: 10.1038/npp.2011.319
Takeuchi, H., Mizoguchi, H., Doi, Y., Jin, S., Noda, M., Liang, J., ... Suzumura, A. (2012). Blockade of gap junction hemichannel suppresses disease progression in mouse models of amyotrophic lateral sclerosis and Alzheimer's disease. PLoS One, 6(6), e21108.
Takeuchi, H., & Suzumura, A. (2014). Gap junctions and hemichannels composed of connexins: potential therapeutic targets for neurodegenerative diseases. Front Cell Neurosci, 8, 189. doi: 10.3389/fncel.2014.00189
Tong, X., Dong, S., Yu, M., Wang, Q., & Tao, L. (2013). Role of heteromeric gap junctions in the cytotoxicity of cisplatin. Toxicology. doi: 10.1016/j.tox.2013.05.010
van Enkhuizen, J., Milienne-Petiot, M., Geyer, M. A., & Young, J. W. (2015). Modeling bipolar disorder in mice by increasing acetylcholine or dopamine: chronic lithium treats most, but not all features. Psychopharmacology (Berl). doi: 10.1007/s00213-015-4000-4
Vannucci, R. C. (2000). Hypoxic-ischemic encephalopathy. Am J Perinatol, 17(3), 113-120. doi: 10.1055/s-2000-9293
Veauthier, C. (2015). Sleep disorders in multiple sclerosis. Review. Curr Neurol Neurosci Rep, 15(5), 21. doi: 10.1007/s11910-015-0546-0
Vega, J. L., Subiabre, M., Figueroa, F., Schalper, K. A., Osorio, L., Gonzalez, J., & Saez, J. C. (2013). Role of gap junctions and hemichannels in parasitic infections. Biomed Res Int, 2013, 589130. doi: 10.1155/2013/589130
Wang, S. (2012). Juvenile neuronal ceroid lipofuscinoses. Adv Exp Med Biol, 724, 138-142. doi: 10.1007/978-1-4614-0653-2_11
Weinstock, M., Gorodetsky, E., Poltyrev, T., Gross, A., Sagi, Y., & Youdim, M. (2003). A novel cholinesterase and brain-selective monoamine oxidase inhibitor for the treatment of dementia comorbid with depression and Parkinson's disease. Prog Neuropsychopharmacol Biol Psychiatry, 27(4), 555-561. doi: 10.1016/S0278-5846(03)00053-8
Wooltorton, J. R., & Mathie, A. (1995). Potent block of potassium currents in rat isolated sympathetic neurones by the uncharged form of amitriptyline and related tricyclic compounds. Br J Pharmacol, 116(4), 2191-2200.
Xu, Q., Cheong, Y. K., Yang, F., Tiwari, V., Li, J., Liu, J., Guan, Y. (2014). Intrathecal carbenoxolone inhibits neuropathic pain and spinal wide-dynamic range neuronal activity in rats after an L5 spinal nerve injury. Neurosci Lett, 563, 45-50.
Yankelevitch-Yahav, R., Franko, M., Huly, A., & Doron, R. (2015). The forced swim test as a model of depressive-like behavior. J Vis Exp(97). doi: 10.3791/52587
Zhang, J., O'Carroll, S. J., Henare, K., Ching, L. M., Ormonde, S., Nicholson, L. F., Green, C. R. (2014). Connexin hemichannel induced vascular leak suggests a new paradigm for cancer therapy. FEBS Lett, 588(8), 1365-1371. doi: 10.1016/j.febslet.2014.02.003

## Claims

1. A therapeutic composition comprising amitriptyline and mefloquine, or any pharmaceutically acceptable salt thereof.

2. The therapeutic composition according to claim 1, for use for preventing and/or treating Major depressive disorder (MDD), post-traumatic stress disorder (PTSD), anxiety, generalized anxiety disorder (GAD), social anxiety disorder (SAD), panic disorder, neuropathic pain, resistant pain, fibromyalgia, chronic musculoskeletal pain, akynesia in Parkinson's disease, cataplexy, migraine, Parkinson's disease, vasomotor symptoms of menopause, premenstrual dysphoric disorder (PMDD), restless syndrome, hypersalivation, hypersalivation in amyotrophic lateral sclerosis, bipolar I disorder, bulimia, obsessive-compulsive disorder (OCD), Alzheimer's disease, Huntington's disease, multiple sclerosis, amyotrophic lateral sclerosis, dementia with Lewy bodies, multiple system atrophy, stress, depression, depression in Parkinson disease and in dementia with Lewy bodies, schizophrenia, autism, insomnia, nocturnal enuresis, nocturnal terrors, sleep bruxism, sleep disorders in fibromyalgia, breathing disorders, REM sleep disorders, sleep apnea, or fibrositis, in a subject in need thereof.

3. The therapeutic composition according to claim 1, for use for preventing and/or treating major depressive disorders, neuropathic pain, anxiety, fibromyalgia, vasomotor symptoms of menopause, or nocturnal enuresis.

4. The therapeutic composition according to claim 1, for use for preventing and/or treating post herpetic neuropathic pain, diabetic neuropathic pain, post chirurgical neuropathic pain, post chemotherapy neuropathic pain, post-stroke neuropathic pain, post cancer treatment neuropathic pain or post chemoprophylaxis neuropathic pain, post-HIV neuropathic pain, chronic back neuropathic pain, low back neuropathic pain, traumatic neuropathy, neuropathic pain related to multiple sclerosis disease or to other immune diseases, or neuropathic pain induced by spinal cord injury.

5. The therapeutic composition according to claim 1 for use according to any one of claims 2 to 4, wherein mefloquine dose regimen is within the range of about 10 µg to 50 mg per day, preferably between 100 µg and 25 mg per day, more preferably between 1 mg and 25 mg per day.

6. The therapeutic composition according to claim 1 for use according to any one of claims 2 to 5, wherein amitriptyline dose regimen is between 10 µg and 150 mg per day, preferably between 1 mg and 75 mg per day, more preferably between 1 mg and 50 mg per day.

7. The therapeutic composition according to claim 1 for use according to any one of claims 2 to 6, wherein it is a tablet, a drinkable solution, a topical cream or a patch.

8. A combination product comprising amitriptyline and mefloquine or any pharmaceutically acceptable salt thereof, for simultaneous, separated, or staggered use for preventing and/or treating Major depressive disorder (MDD), post-traumatic stress disorder (PTSD), anxiety, generalized anxiety disorder (GAD), social anxiety disorder (SAD), panic disorder, neuropathic pain, resistant pain, fibromyalgia, chronic musculoskeletal pain, akynesia in Parkinson's disease, cataplexy, migraine, Parkinson's disease, vasomotor symptoms of menopause, premenstrual dysphoric disorder (PMDD), restless syndrome, hypersalivation, hypersalivation in amyotrophic lateral sclerosis, bipolar I disorder, bulimia, obsessive-compulsive disorder (OCD), Alzheimer's disease, Huntington's disease, multiple sclerosis, amyotrophic lateral sclerosis, dementia with Lewy bodies, multiple system atrophy, stress, depression, depression in Parkinson disease and in dementia with Lewy bodies, schizophrenia, autism, insomnia, nocturnal enuresis, nocturnal terrors, sleep bruxism, sleep disorders in fibromyalgia, breathing disorders, REM sleep disorders, sleep apnea, or fibrositis, in a subject in need thereof.

9. The combination product of claim 8, for simultaneous, separated, or staggered use for preventing and/or treating major depressive disorders, neuropathic pain, anxiety, fibromyalgia, vasomotor symptoms of menopause, or nocturnal enuresis.

10. The combination product of claim 8, for simultaneous, separated, or staggered use for preventing and/or treating post herpetic neuropathic pain, diabetic neuropathic pain, post chirurgical neuropathic pain, post chemotherapy neuropathic pain, post-stroke neuropathic pain, post cancer treatment neuropathic pain or post chemoprophylaxis neuropathic pain, post-HIV neuropathic pain, chronic back neuropathic pain, low back neuropathic pain, traumatic neuropathy, neuropathic pain related to multiple sclerosis disease or to other immune diseases, or neuropathic pain induced by spinal cord injury.

11. The combination product for simultaneous, separated, or staggered use of any of claims 8 to 10, wherein mefloquine dose regimen is within the range of about 10 µg to 50 mg per day, preferably from 100 µg to 25 mg per day, more preferably between 1 mg and 25 mg per day.

12. The combination product for simultaneous, separated, or staggered use of any of claims 8 to 10, wherein amitriptyline dose regimen is between 10 µg and 150 mg per day, preferably between 1 mg and 75 mg per day, more preferably between 1 mg and 50 mg per day.

## Patentansprüche

1. Therapeutische Zusammensetzung, die Amitriptylin und Mefloquin oder ein beliebiges pharmazeutisch annehmbares Salz davon umfasst.

2. Therapeutische Zusammensetzung nach Anspruch 1 zur Verwendung für die Prävention und/oder Behandlung von Depression (Major Depressive Disorder - MDD), Posttraumatischer Belastungsstörung (Post-Traumatic Stress Disorder - PTSD), Angstzuständen, generalisierter Angsstörung (Generalized Anxiety Disorder - GAD), Sozialer Angststörung (Social Anxiety Disorder - SAD), Panikstörung, neuropathischem Schmerz, resistentem Schmerz, Fibromyalgie, chronischem muskuloskelettalem Schmerz, Akinese bei Parkinson-Krankheit, Kataplexie, Migräne, Parkinson-Krankheit, vasomotorischen Symptomen in der Menopause, premenstrueller dysphorischer Störung (Premenstrual Dysphoric Disorder - PMDD), Restless-Syndrom, Hypersalivation, Hypersalivation bei amyotropher Lateralsklerose, bipolarer Störung I, Bulimie, Zwangsstörung (Obsessive-Compulsive Disorder - OCD), Alzheimer-Krankheit, Huntington-Krankheit, multipler Sklerose, amyotropher Lateralsklerose, Lewy-Körper-Demenz, multipler Systematrophie, Stress, Depression, Depression bei Parkinson-Krankheit und bei Lewy-Körper-Demenz, Schizophrenie, Autismus, Schlafstörungen/Insomnie, Bettnässen (Enuresis nocturna), Pavor nocturnus (Nachtangst), Bruxismus (nächtlichem Zähneknirschen), Schlafstörungen bei Fibromyalgie, Atemstörungen, REM-Schlafstörungen, Schlafapnoe oder Fibrositis bei einem Subjekt, das diese benötigt.

3. Therapeutische Zusammensetzung nach Anspruch 1 zur Verwendung für die Prävention und/oder Behandlung von Depression (Major Depressive Disorder - MDD), neuropathischem Schmerz, Angstzuständen, Fibromyalgie, vasomotorischen Symptomen in der Menopause oder Bettnässen (Enuresis nocturna).

4. Therapeutische Zusammensetzung nach Anspruch 1 zur Verwendung für die Prävention und/oder Behandlung von postherpetischem neuropathischem Schmerz, diabetischem neuropathischem Schmerz, postoperativem neuropathischem Schmerz, neuropathischem Schmerz nach Chemotherapie, neuropathischem Schmerz nach Schlaganfall, neuropathischem Schmerz nach Krebsbehandlung oder neuropathischem Schmerz nach Chemoprophylaxe, neuropathischem Schmerz nach HIV-Erkrankung, chronischem neuropathischem Rückenschmerz, chronischem neuropathischem Schmerz im unteren Rücken, traumatischer Neuropathie, neuropathischem Schmerz in Verbindung mit Multipler-Sklerose-Erkrankung oder mit anderen Immunerkrankungen oder durch Rückenmarksverletzungen induziertem neuropathischem Schmerz.

5. Therapeutische Zusammensetzung nach Anspruch 1 zur Verwendung nach einem der Ansprüche 2 bis 4, wobei das Mefloquin-Dosierungsschema im Bereich von etwa 10 µg bis 50 mg pro Tag, vorzugsweise zwischen 100 µg und 25 mg pro Tag, mehr zu bevorzugen zwischen 1 mg und 25 mg pro Tag, liegt.

6. Therapeutische Zusammensetzung nach Anspruch 1 zur Verwendung nach einem der Ansprüche 2 bis 5, wobei das Amitriptylin-Dosierungsschema zwischen 10 µg und 150 mg pro Tag, vorzugsweise zwischen 1 mg und 75 mg, mehr zu bevorzugen zwischen 1 mg bis 50 mg pro Tag beträgt.

7. Therapeutische Zusammensetzung nach Anspruch 1 zur Verwendung nach einem der Ansprüche 2 bis 6, wobei sie eine Tablette, eine trinkbare Lösung, eine topische Creme oder ein Pflaster ist.

8. Kombinationsprodukt, das Amitriptylin und Mefloquin oder ein beliebiges pharmazeutisch annehmbares Salz davon umfasst, zur gleichzeitigen, getrennten oder gestaffelten Verwendung für die Prävention und/oder Behandlung von Depression (Major Depressive Disorder - MDD), Posttraumatischer Belastungsstörung (Post-Traumatic Stress Disorder - PTSD), Angstzuständen, generalisierter Angsstörung (Generalized Anxiety Disorder - GAD), Sozialer Angststörung (Social Anxiety Disorder - SAD), Panikstörung, neuropathischem Schmerz, resistentem Schmerz, Fibromyalgie, chronischem muskuloskelettalem Schmerz, Akinese bei Parkinson-Krankheit, Kataplexie, Migräne, Parkinson-Krankheit, vasomotorischen Symptomen in der Menopause, premenstrueller dysphorischer Störung (Premenstrual Dysphoric Disorder - PMDD), Restless-Syndrom, Hypersalivation, Hypersalivation bei amyotropher Lateralsklerose, bipolarer Störung I, Bulimie, Zwangsstörung (Obsessive-Compulsive Disorder - OCD), Alzheimer-Krankheit, Huntington-Krankheit, multipler Sklerose, amyotropher Lateralsklerose, Lewy-Körper-Demenz, multipler Systematrophie, Stress, Depression, Depression bei Parkinson-Krankheit und bei Lewy-Körper-Demenz, Schizophrenie, Autismus, Schlafstörungen/Insomnie, Bettnässen (Enuresis nocturna), Pavor nocturnus (Nachtangst), Bruxismus (nächtlichem Zähneknirschen), Schlafstörungen bei Fibromyalgie, Atemstörungen, REM-Schlafstörungen, Schlafapnoe oder Fibrositis bei einem Subjekt, das diese benötigt.

9. Kombinationsprodukt nach Anspruch 8 zur gleichzeitigen, getrennten oder gestaffelten Verwendung für die Prävention und/oder Behandlung von Depression (Major Depressive Disorder - MDD), neuropathischem Schmerz, Angstzuständen, Fibromyalgie, vasomotorischen Symptomen in der Menopause oder Bettnässen (Enuresis nocturna).

10. Kombinationsprodukt nach Anspruch 8 zur gleichzeitigen, getrennten oder gestaffelten Verwendung für die Prävention und/oder Behandlung von postherpetischem neuropathischem Schmerz, diabetischem neuropathischem Schmerz, postoperativem neuropathischem Schmerz, neuropathischem Schmerz nach Chemotherapie, neuropathischem Schmerz nach Schlaganfall, neuropathischem Schmerz nach Krebsbehandlung oder neuropathischem Schmerz nach Chemoprophylaxe, neuropathischem Schmerz nach HIV-Erkrankung, chronischem neuropathischem Rückenschmerz, chronischem neuropathischem Schmerz im unteren Rücken, traumatischer Neuropathie, neuropathischem Schmerz in Verbindung mit Multipler-Sklerose-Erkrankung oder mit anderen Immunerkrankungen oder durch Rückenmarksverletzungen induziertem neuropathischem Schmerz.

11. Kombinationsprodukt zur gleichzeitigen, getrennten oder gestaffelten Verwendung nach einem der Ansprüche 8 bis 10, wobei das Mefloquin-Dosierungsschema im Bereich von etwa 10 µg bis 50 mg pro Tag, vorzugsweise von 100 µg bis 25 mg pro Tag, mehr zu bevorzugen zwischen 1 mg und 25 mg pro Tag, liegt.

12. Kombinationsprodukt zur gleichzeitigen, getrennten oder gestaffelten Verwendung nach einem der Ansprüche 8 bis 10, wobei das Amitriptylin-Dosierungsschema zwischen 10 µg und 150 mg pro Tag, vorzugsweise zwischen 1 mg und 75 mg pro Tag, mehr zu bevorzugen zwischen 1 mg bis 50 mg pro Tag, beträgt.

## Revendications

1. Composition thérapeutique comprenant de l'amitriptyline et de la méfloquine, ou n'importe quels sels pharmaceutiquement acceptables de celles-ci.

2. Composition thérapeutique selon la revendication 1, pour une utilisation afin de prévenir et/ou de traiter un trouble dépressif majeur (TDM), un trouble de stress post-traumatique (TSPT), une anxiété, un trouble de l'anxiété généralisée (TAG), un trouble de l'anxiété sociale (TAS), un trouble panique, une douleur neuropathique, une douleur résistante, une fibromyalgie, une douleur musculo-squelettique chronique, une akynésie lors de la maladie de Parkinson, une cataplexie, une migraine, la maladie de Parkinson, les symptômes vasomoteurs de la ménopause, un trouble dysphorique prémenstruel (TDPM), un syndrome des jambes sans repos, une hypersalivation, une hypersalivation lors d'une sclérose latérale amyotrophique, un trouble bipolaire de type I, une boulimie, un trouble obsessionnel compulsif (TOC), la maladie d'Alzheimer, la chorée de Huntington, la sclérose en plaques, la sclérose latérale amyotrophique, une démence à corps de Lewy, une atrophie multisystématisée, un stress, une dépression, une dépression lors de la maladie de Parkinson et lors d'une démence à corps de Lewy, une schizophrénie, un autisme, une insomnie, une énurésie nocturne, des terreurs nocturnes, un bruxisme nocturne, des troubles du sommeil lors d'une fibromyalgie, des troubles respiratoires, des troubles du sommeil paradoxal, une apnée du sommeil, ou une fibrosite, chez un sujet en ayant besoin.

3. Composition thérapeutique selon la revendication 1, pour une utilisation afin de prévenir et/ou de traiter des troubles dépressifs majeurs, une douleur neuropathique, une anxiété, une fibromyalgie, des symptômes vasomoteurs de la ménopause, ou une énurésie nocturne.

4. Composition thérapeutique selon la revendication 1, pour une utilisation afin de prévenir et/ou de traiter une douleur neuropathique post-herpétique, une douleur neuropathique diabétique, une douleur neuropathique post-chirurgicale, une douleur neuropathique post-chimiothérapie, une douleur neuropathique post-accident vasculaire, une douleur neuropathique post-traitement anticancéreux ou une douleur neuropathique post-chimioprophylaxie, une douleur neuropathique post-VIH, une douleur neuropathique chronique du dos, une douleur neuropathique lombaire, une neuropathie traumatique, une douleur neuropathique liée à une sclérose en plaques ou à d'autres maladies immunitaires, ou une douleur neuropathique induite par une lésion de la moelle épinière.

5. Composition thérapeutique selon la revendication 1 pour une utilisation selon l'une quelconque des revendications 2 à 4, dans laquelle le régime posologique de la méfloquine est situé dans la plage allant d'environ 10 µg à 50 mg par jour, de préférence compris entre 100 µg et 25 mg par jour, mieux encore compris entre 1 mg et 25 mg par jour.

6. Composition thérapeutique selon la revendication 1 pour une utilisation selon l'une quelconque des revendications 2 à 5, dans laquelle le régime posologique de l'amitriptyline est compris entre 10 µg et 150 mg par jour, de préférence entre 1 mg et 75 mg par jour, mieux encore entre 1 mg et 50 mg par jour.

7. Composition thérapeutique selon la revendication 1 pour une utilisation selon l'une quelconque des revendications 2 à 6, qui est un comprimé, une solution buvable, une crème à usage topique ou un patch transdermique.

8. Produit combiné comprenant de l'amitriptyline et de la méfloquine, ou n'importe quels sels pharmaceutiquement acceptables de celles-ci, pour une utilisation simultanée, séparée ou échelonnée afin de prévenir et/ou de traiter un trouble dépressif majeur (TDM), un trouble de stress post-traumatique (TSPT), une anxiété, un trouble de l'anxiété généralisée (TAG), un trouble de l'anxiété sociale (TAS), un trouble panique, une douleur neuropathique, une douleur résistante, une fibromyalgie, une douleur musculo-squelettique chronique, une akynésie lors de la maladie de Parkinson, une cataplexie, une migraine, la maladie de Parkinson, les symptômes vasomoteurs de la ménopause, un trouble dysphorique prémenstruel (TDPM), un syndrome des jambes sans repos, une hypersalivation, une hypersalivation lors d'une sclérose latérale amyotrophique, un trouble bipolaire de type I, une boulimie, un trouble obsessionnel compulsif (TOC), la maladie d'Alzheimer, la chorée de Huntington, la sclérose en plaques, la sclérose latérale amyotrophique, une démence à corps de Lewy, une atrophie multisystématisée, un stress, une dépression, une dépression lors de la maladie de Parkinson et lors d'une démence à corps de Lewy, une schizophrénie, un autisme, une insomnie, une énurésie nocturne, des terreurs nocturnes, un bruxisme nocturne, des troubles du sommeil lors d'une fibromyalgie, des troubles respiratoires, des troubles du sommeil paradoxal, une apnée du sommeil, ou une fibrosite, chez un sujet en ayant besoin.

9. Produit combiné selon la revendication 8, pour une utilisation simultanée, séparée ou échelonnée afin de prévenir et/ou de traiter des troubles dépressifs majeurs, une douleur neuropathique, une anxiété, une fibromyalgie, des symptômes vasomoteurs de la ménopause, ou une énurésie nocturne.

10. Produit combiné selon la revendication 8, pour une utilisation simultanée, séparée ou échelonnée afin de prévenir et/ou de traiter une douleur neuropathique post-herpétique, une douleur neuropathique diabétique, une douleur neuropathique post-chirurgicale, une douleur neuropathique post-chimiothérapie, une douleur neuropathique post-accident vasculaire, une douleur neuropathique post-traitement anticancéreux ou une douleur neuropathique post-chimioprophylaxie, une douleur neuropathique post-VIH, une douleur neuropathique chronique du dos, une douleur neuropathique lombaire, une neuropathie traumatique, une douleur neuropathique liée à une sclérose en plaques ou à d'autres maladies immunitaires, ou une douleur neuropathique induite par une lésion de la moelle épinière.

11. Produit combiné pour une utilisation simultanée, séparée ou échelonnée selon l'une quelconque des revendications 8 à 10, dans lequel le régime posologique de la méfloquine est situé dans la plage allant d'environ 10 µg à 50 mg par jour, de préférence compris entre 100 µg et 25 mg par jour, mieux encore compris entre 1 mg et 25 mg par jour.

12. Produit combiné pour une utilisation simultanée, séparée ou échelonnée selon l'une quelconque des revendications 8 à 10, dans lequel le régime posologique de l'amitriptyline est compris entre 10 µg et 150 mg par jour, de préférence entre 1 mg et 75 mg par jour, mieux encore entre 1 mg et 50 mg par jour.
